(19) Europäisches Patentamt
European Patent Office
Office européen des brevets

(11) **EP 1 265 843 B1**

(12) **EUROPÄISCHE PATENTSCHRIFT**

(45) Veröffentlichungstag und Bekanntmachung des Hinweises auf die Patenterteilung:
**09.11.2005 Patentblatt 2005/45**

(21) Anmeldenummer: **01925468.9**

(22) Anmeldetag: **15.03.2001**

(51) Int Cl.[7]: **C07C 69/80**, C07C 69/60, C14C 9/02, B01F 17/00, C07C 67/08, C14C 9/00, C07C 303/04, C07C 309/22

(86) Internationale Anmeldenummer:
**PCT/EP2001/002952**

(87) Internationale Veröffentlichungsnummer:
**WO 2001/068584 (20.09.2001 Gazette 2001/38)**

(54) **MISCHUNGEN VON HALBESTERN MEHRBASIGER ORGANISCHER SÄUREN UND LANGKETTIGER ALKANOLE,IHRE HERSTELLUNG UND VERWENDUNG**

MIXTURES OF SEMI-ESTERS OF POLYBASIC ORGANIC ACIDS AND LONG-CHAIN ALKANOLS, THE PRODUCTION AND THE USE THEREOF

MELANGES DE SEMI-ESTERS D'ACIDES ORGANIQUES POLYBASIQUES ET D'ALCANOLS A LONGUE CHAINE, LEUR PRODUCTION ET LEUR UTILISATION

(84) Benannte Vertragsstaaten:
**AT BE CH CY DE DK ES FI FR GB GR IE IT LI LU MC NL PT SE TR**

(30) Priorität: **16.03.2000 DE 10012722**

(43) Veröffentlichungstag der Anmeldung:
**18.12.2002 Patentblatt 2002/51**

(73) Patentinhaber: **BASF Aktiengesellschaft**
**67056 Ludwigshafen (DE)**

(72) Erfinder:
- **WEYLAND, Peter**
  **67227 Frankenthal (DE)**
- **DEMHARTER, Susanne**
  **68199 Mannheim (DE)**
- **DANISCH, Peter**
  **67065 Ludwigshafen (DE)**
- **OETTER, Günter**
  **67227 Frankenthal (DE)**

(74) Vertreter: **Isenbruck, Günter et al**
**Isenbruck, Bösl, Hörschler, Wichmann, Huhn**
**Patentanwälte**
**Theodor-Heuss-Anlage 12**
**68165 Mannheim (DE)**

(56) Entgegenhaltungen:
**DE-A- 1 669 347** **DE-A- 3 507 241**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 265 843 B1

Printed by Jouve, 75001 PARIS (FR)

## Beschreibung

Die Erfindung betrifft komplexe Mischungen von Halbestern aus mehrbasigen organischen Säuren und langkettigen Alkanolen, Alkoxyalkanolen und Diolen, insbesondere Dicklölen aus der Oxosynthese, ihre Herstellung und ihre Verwendung als preisgünstige, gut wirksame Emulgatoren, und insbesondere als Hilfsmittel bei der Lederherstellung.

Bei der Herstellung von Leder werden neben den eigentlichen organischen und/oder anorganischen Gerbstoffen zahlreiche Hilfsmittel eingesetzt, die die Eigenschaften des Leders, insbesondere seine Schmiegsamkeit, seinen Griff und sein Gebrauchsverhalten, insbesondere seine Wasseraufnahme und durchlässigkeit wesentlich mitbestimmen.

Eine wesentliche Gruppe derartiger Hilfsmittel sind die sogenannten fettenden Ausrüstungsmittel, die, in das Leder eingelagert, die Verhärtung des Leders verhindern und in speziellen Ausführungsformen als Hydrophobiermittel seine Wasserfestigkeit verbessern sollen. Der durch die Fettungs- bzw. Hydrophobiermittel erzielte Effekt soll möglichst dauerhaft sein und auch Behandlungen des ausgerüsteten Leders mit Wasser, wäßrigen Tensidlösungen und ggf. auch Mitteln zur chemischen Reinigung widerstehen.

Aus diesen Forderungen folgt, daß ein Fettungs- bzw. Hydrophobiermittel gut in das Gefüge des Leders eindringen können, es gut abdichten und erweichen und darin wasch- und reinigungsfest verankert werden muß, wenn es seinen Zweck in ausreichendem Maß erfüllen soll. Eine weitere, wesentliche Forderung ist, daß die ausgerüsteten Ledermaterialien keine schmierige Oberfläche aufweisen sollen.

Aus der DE 16 69 347 ist die Fettung von Leder mit wasseremulgierbaren Sulfobernsteinsäure-Halbestern bekannt. Die so behandelten Leder sind jedoch nicht wasserdicht.

Aus der EP-A-193 832 ist es bekannt, wasserdichte Leder durch eine Behandlung des Leders mit einer Kombination von wasseremulgierbaren Sulfobernsteinsäure-Halbestern mit imprägnierenden und/oder hydrophobierenden Fettungsmitteln und anschließende Fixierung mit Al-, Cr- oder Zr-Salzen herzustellen.

In der EP-A-372 746 ist für die Fettung von Leder der Einsatz von Copolymeren aus einem überwiegenden Anteil hydrophober und einem untergeordneten Anteil hydrophiler Monomerer und in der EP-A-412 389 für den gleichen Zweck die Verwendung von Carboxylgruppen enthaltenden Copolymerisaten beschrieben worden.

Einem ähnlichen Prinzip folgt das aus der DE-A-41 29 244 bekannte Fettungsverfahren: Hier werden als Wirksubstanz wäßrige Dispersionen Oligomerer eingesetzt, die Carboxylgruppen, Estergruppen und ggf. auch Polyetherketten aufweisen.

In der DE-A-196 44 242 wird der Einsatz von Umsetzungsprodukten von langkettigen aliphatischen Monocarbonsäure, z.B. Stearinsäure, mit niedermolekularen aliphatischen Hydroxy-polycarbonsäuren, z.B. Zitronensäure, und von Umsetzungsprodukten von Fettalkoholen, z.B. Stearylalkohol, mit Pyromellithsäuredianhydrid im Molverhältnis von etwa 1:2 zur Lederfettung empfohlen. Die festen Umsetzungsprodukte werden unter Einsatz von Emulgatoren in cremeartige Pasten überführt. Sie liefern gute Werte der Wasserdurchtrittszeiten, sind aber aufgrund ihrer pastösen Konsistenz umständlich zu applizieren. Ein weiterer Nachteil ist, daß sie aus relativ teuren Ausgangsmaterialien hergestellt werden müssen.

Aus der DE-A-44 05 205 ist die Verwendung von wasserdispergierbaren Partialestern aus mehrbasigen, vorzugsweise drei- oder vierbasigen cyclischen Carbonsäuren mit monofunktionellen Fettalkoholen exakt definierter Kettenlänge zur Lederfettung bekannt. Beispielsweise wird ein Partialester, hergestellt aus Pyromellithsäuredianhydrid und einem gesättigten $C_{18}$-Fettalkohol im Molverhältnis 1:2 eingesetzt.

Als wichtige Eigenschaften dieser Mittel werden einerseits die sichere Fixierung der amphiphilen Substanzen über die freien Carboxylgruppen genannt, andererseits die genaue Einstellung der Penetration in das Leder ohne die Ausbildung einer schmierigen Oberfläche. Um diese Eigenschaften zu erreichen muß bei dieser Methode allerdings das Molekulargewicht der Partialester sehr exakt eingestellt werden, wozu der Einsatz unverhältnismäßig teurer Fettalkohole definierter Kettenlänge und Struktur erforderlich ist. Außerdem wird nach Einsatz dieser Produkte eine Fixierung durch eine Metallsalzbehandlung, insbesondere eine Chromgerbung empfohlen. Die hier eingesetzten Produkte liegen in der Regel als cremeartige bis feste Dispersionen vor, was die Anwendung erschwert.

Die in der Einleitung erwähnte Oxosynthese ist ein technisch in großem Umfang ausgeführter Prozeß zur Herstellung von Aldehyden und Alkoholen. Bei diesem Prozeß wird in Gegenwart geeigneter Katalysatoren an die Doppelbindung von Olefinen Kohlenmonoxid und Wasserstoff angelagert. Der Oxoprozeß eignet sich zur Herstellung eines sehr breiten Spektrums technisch interessanter aliphatischer Substanzen mit Kohlenstoffketten sehr unterschiedlicher Länge. Er ist wegen seiner großen technischen Bedeutung in vielen Richtungen bearbeitet und modifiziert worden. Eine gute Übersicht über die Technologie des Oxoprozesses findet sich beispielsweise bei B. Cornils, in J.Falbe. *New Syntheses with Carbon-Monoxide,* Springer-Verlag, Berlin (1980), *Ullmanns Encyklopädie der technischen Chemie.* 4.Aufl. Bd.7. Seiten 118 ff.. und *Winnacker-Küchler*, 4.Aufl Bd. 5, Seiten 537 ff..

In einer wichtigen Ausführungsform, bei der phosphinmodifizierte Kobaltkatalysatoren eingesetzt werden, gelingt es, direkt wertvolle langkettige Alkohole in hohen Ausbeuten herzustellen (*Winnacker-Küchler*, 4.Aufl. Bd. 5,

Seiten 537 ff.).

Wie bei jedem technisch durchgeführten Prozeß kommt es auch bei der Oxosynthese aufgrund parallel laufender Nebenreaktionen und Folgereaktionen zur Bildung von Nebenprodukten, die bei einer Ausführungsform des Prozesses, die auf die Herstellung langkettiger Alkohole gerichtet ist, im Wesentlichen eine Mischung höhermolekularer Alkohole, Ether, Ester und Diole darstellen.

Diese Mischung, in der Literatur häufig als "Dicköl" bezeichnet, wird bei der Destillation des rohen Oxoproduktes als Sumpfprodukt erhalten. Dieses Dicköl, - im Folgenden zur eindeutigen Abgrenzung gegen Dicköle anderer Art als "Oxodicköl" bezeichnet - wird zur Zeit nur nach seinem Brennwert bewertet und stellt somit eine Belastung des Oxoprozesses dar.

Es wurde nun überraschend gefunden, daß komplexe Mischungen von amphiphilen Halbestern mehrbasiger Säuren und höhermolekularer Alkanole, die sich ausgezeichnet als Emulgatoren, insbesondere für Lederfettungsmittel und Hydrophobiermittel eignen, unter Einsatz der Oxodicköle preisgünstig hergestellt werden können.

Die vorliegende Erfindung betrifft somit sowohl die komplexen Mischungen von amphiphilen Halbestern mehrbasiger Säuren und höhermolekularer Alkanole, die sich ausgezeichnet als Emulgatoren, insbesondere für Lederfettungsmittel und Hydrophobiermittel eignen, und vorzugsweise solcher, die als Alkoholkomponenten die Bestandteile von Oxodickölen aufweisen, als auch deren Herstellung, Zubereitungen dieser Halbestermischungen und deren Verwendung als Emulgatoren für Fettungs- und Hydrophobiermittel.

In erster Linie betrifft die vorliegende Erfindung Gemische von Monoestern zwei- oder dreibasiger Carbonsäuren der Formeln I und II,

$$(MOOC)_a\text{-}R^1\text{-CO-OR}^2 \qquad \text{(I)}$$

$$[(MOOC)_a\text{-}R^1\text{-CO-O}]_2R^3 \qquad \text{(II)}$$

worin a die Ziffern 1 oder 2 bedeutet, M für Wasserstoff oder ein Metalläquivalent steht und

- $R^1$ ein zwei- oder dreiwertiger, gesättigter oder ein- oder zweifach ungesättigter aliphatischer oder cycloaliphatischer Kohlenwasserstoffrest mit 2 bis 6 C-Atomen, der durch eine Sulfonsäuregruppe substituiert sein kann, oder ein zwei- oder dreiwertiger aromatischer Kohlenwasserstoffrest mit 6 C-Atomen,
- $R^2$ überwiegend verzweigtes, gegebenenfalls durch Hydroxy, Alkoxy. Alkylcarbonyloxy oder Alkoxycarbonyl substituiertes Alkyl mit 9 bis 51, vorzugsweise 9 bis 45 C-Atomen und
- $R^3$ Alkandiyl mit 10 bis 30 C-Atomen bedeutet,

mit von OH-Gruppen freien Ethern und Estern mit 18 bis 45 C-Atomen und gegebenenfalls Alkanolen der Formel $R^2OH$ und Alkandiolen der Formel $R^3(OH)_2$,
wobei, - bezogen auf die Summe SUG der Gewichte der in der Mischung enthaltenen von OH-Gruppen freien Ether, von OH-Gruppen freien Ester, Alkanole, Alkandiole und $-OR^2$- und $(-O)_2R^3$-Gruppen, - der Anteil der $-OR^2$-Gruppen bis zu 85 %, der Anteil der $(-O)_2R^3$-Gruppen bis zu 16 %, und der Anteil der von OH-Gruppen freien Ether und Ester bis zu 45 % beträgt.

Es ist zu beachten, daß der oben angegebene Anteil der $-OR^2$-Gruppen und $(-O)_2R^3$-Gruppen alle derartigen Gruppen umfaßt, unabhängig davon, in welchem Mischungsbestandteil sie enthalten sind, also sowohl die in den Estern der Formeln I und II enthaltenen Gruppen als auch die in gegebenenfalls vorhandenen Alkanolen und Alkandiolen enthaltenen $-OR^2$-Gruppen und $(-O)_2R^3$-Gruppen einschließt.

Analog gilt für die Bezugsgröße SUG, daß bei ihrer Berechnung ebenfalls das Gewicht aller $-OR^2$-Gruppen und $(-O)_2R^3$-Gruppen, unabhängig davon, in welchem Mischungsbestandteil sie enthalten sind, eingesetzt wird.

In einer bevorzugten Ausführungsform der Erfindung liegt der Anteil der Alkanole der Formel $R^2OH$ und Alkandiole der Formel $R^3(OH)_2$, bezogen auf das Gesamtgewicht der Mischung, unter 5 Gew.%, vorzugsweise unter 2 Gew.-% insbesondere unter 0,5 Gew.-%.

In einer weiteren bevorzugten Ausführungsform der Erfindung beträgt der Anteil der Alkanole der Formel $R^2OH$ und Alkandiole der Formel $R^3(OH)_2$, bezogen auf das Gesamtgewicht der Mischung, bis zu 65 Gew.-%, vorzugsweise bis zu 60 Gew.-%, insbesondere bis zu 55 Gew.-%.

Bevorzugt sind erfindungsgemäße Gemische von Monoestern zwei- oder dreibasiger Carbonsäuren der Formeln I und II,

$$(MOOC)_a\text{-}R^1\text{-}CO\text{-}OR^2 \quad (I)$$

$$[(MOOC)_a\text{-}R^1\text{-}CO\text{-}O]_2R^3 \quad (II)$$

worin a die Ziffern 1 oder 2 bedeutet, M für Wasserstoff oder ein Metalläquivalent steht und

$R^1$ ein zweiwertiger, gesättigter aliphatischer Kohlenwasserstoffrest mit 2 bis 4 C-Atomen, der durch eine Sulfonsäuregruppe substituiert sein kann, oder ein zweiwertiger, einfach ungesättigter aliphatischer Kohlenwasserstoffrest mit 2 bis 4 C-Atomen,
oder ein zweiwertiger oder dreiwertiger, gesättigter cycloaliphatischer Kohlenwasserstoffrest mit 6 C-Atomen, der durch eine Sulfonsäuregruppe substituiert sein kann
oder ein zwei- oder dreiwertiger cycloaliphatischer Kohlenwasserstoffrest mit 6 C-Atomen, der eine oder ggf. zwei Doppelbindungen aufweist,
oder ein zwei- oder dreiwertiger aromatischer Kohlenwasserstoffrest mit 6 C-Atomen bedeutet,

$R^2$ die Reste $R^{2A}$, $R^{2B}$, $R^{2C}$, $R^{2D}$ und $R^{2E}$ umfaßt, wobei

$R^{2A}$ Alkyl(1)- und Alkyl(2)-Reste mit 9 bis 15 C-Atomen, mit einem mittleren Molgewicht von MA
$R^{2B}$ 2-Alkyl-alkyl(1)-Reste mit 18 bis 30 C-Atomen, mit einem mittleren Molgewicht von MB
$R^{2C}$ x-Alkyl-alkyl(y)-Reste mit 18 bis 30 C-Atomen, mit einem mittleren Molgewicht von MC

oder ein zweiwertiger oder dreiwertiger, gesättigter cycloaliphatischer Kohlenwasserstoffrest mit 6 C-Atomen, der durch eine Sulfonsäuregruppe substituiert sein kann
oder ein zwei- oder dreiwertiger cycloaliphatischer Kohlenwasserstoffrest mit 6 C-Atomen, der eine oder ggf. zwei Doppelbindungen aufweist,
oder ein zwei- oder dreiwertiger aromatischer Kohlenwasserstoffrest mit 6 C-Atomen bedeutet,
die Reste $R^{2A}$, $R^{2B}$, $R^{2C}$, $R^{2D}$ und $R^{2E}$ umfasst, wobei

$R^{2A}$ Alkyl(1)- und Alkyl(2)-Reste mit 9 bis 15 C-Atomen, mit einem mittleren Molgewicht von MA
$R^{2B}$ 2-Alkyl-alkyl(1)-Reste mit 18 bis 30 C-Atomen, mit einem mittleren Molgewicht von MB
$R^{2C}$ x-Alkyl-alkyl(y)-Reste mit 18 bis 30 C-Atomen, mit einem mittleren Molgewicht von MC
wobei x die Position der Alkyl-Seitenkette an der Alkyl-Hauptkette und y die Position der Bindungsstelle der Alkyl-Hauptkette an das Sauerstoffatom bedeutet,
$R^{2D}$ Alkoxyalkyl(1)-Reste mit 18 bis 30 C-Atomen, mit einem mittleren Molgewicht von MD
$R^{2E}$ x-Alkylcarbonyloxy-alkyl(y)-Reste und/oder x-Alkoxycarbonyl-alkyl(y)-Reste mit 27 bis 51 C-Atomen mit einem mittleren Molgewicht von ME bedeutet,
wobei x die Position des Alkylcarbonyloxy- bzw. Alkoxycarbonyl-Substituenten an der Alkyl-Hauptkette und y die Position der Bindungsstelle der Alkyl-Hauptkette an das Sauerstoffatom bedeutet,

Die Werte von x und y ergeben sich aus den weiter unten beschriebenen Strukturtypen für die Bestandteile der Reste $-R^2$ und $-R^3$. Vorzugsweise liegt der Zahlenwert von x im Bereich von 9 bis 15, der von y im Bereich von 10 bis 16.

Bevorzugt sind solche erfindungsgemäßen Halbestergemische, bei denen die Anteile der in den Baugruppen $-OR^2$- und $-OR^3$-enthaltenen Strukturen $-OR^{2A}$, $-OR2^{B}$, $-OR^{2C}$, $-OR^{2D}$, $-OR^{2E}$, $-OR^{3F}O$- und $-OR^{3G}O$- und der Anteil der Ether und Ester im Rahmen der oben angegebenen Grenzen so gewählt werden, daß die durch die Gleichung (GL1)

$$OHZ = 561 \cdot \left( \frac{A[\%]}{MA} + \frac{B[\%]}{MB} + \frac{C[\%]}{MC} + \frac{D[\%]}{MD} + \frac{E[\%]}{ME} \right) + 1120 \cdot \left( \frac{F[\%]}{MF} + \frac{G[\%]}{MG} \right) \quad (GL1)$$

worin A[%], B[%], C[%], D[%] E[%], F[%] und G[%] die oben genannten prozentualen Anteile der in den Baugruppen $-OR^2$ und $-OR^3$ enthaltenen Strukturen, bezogen auf das Summengewicht SUG, und MA, MB, MC, MD, ME, MF und MG die Molekulargewichte der besagten Strukturen sind, definierte Größe OHZ im Bereich von 65 bis 160, vorzugsweise im Bereich von 90 bis 140, insbesondere von 90 bis 120 liegt.

Als Molekulargewichte sind die sich aus den Formeln der Reste $-OR^2$ und $-OR^3$ ergebenden Zahlenmittel der Molekulargewichte einzusetzen. Die prozentualen Anteile A[%], B[%], C[%], D[%], E[%], F[%],und G[%] der Mischungsbestandteile müssen sich zu (100-H)[%] ergänzen.

Weiterhin sind solche erfindungsgemäßen Mischungen von Monoestern der Formeln I und II bevorzugt, in denen

$R^1$ Ethan-1,2-diyl, Sulfo-ethan-1.2-diyl, Ethen-1,2-diyl, Propan-1,2-diyl, Propan 1,3-diyl, Sulfopropan-1,2-diyl, Sulfopropan 1,3-diyl, Propen-1,2-diyl, Cyclohexan-1,2-diyl, Sulfocyclohexan-1,2-diyl, Disulfocyclohexan-1,2-diyl, Cyclohex-1-,2-,3- oder 4-en-1,2-diyl, Cyclohex-1,3-, 1,4-. 2,4-oder 2,5-dien-l,2-diyl, Cyclohexan-1,2,4-triyl, Sulfocyclohexan-1.2,4-triyl, Disulfocyclohexan-1,2,4-triyl, Cyclohex-l-,2-,3- oder 4-en-1,2,4-triyl, Cyclohex-1,3-, 1,4-, 2,4- oder 2,5-dien-1,2,4-triyl, Phenylen-1,2, 3-oder 4-Sulfophenylen-1,2 und Phenyl-1,2,4-triyl bedeutet und insbesondere solche in denen

$R^1$ für Ethen-1,2-diyl, Sulfo-ethan-1,2-diyl, Phenylen-1,2, 3- oder 4-Sulfophenylen-1,2 oder Phenyl-1,2,4-triyl steht.

Reste $R^1$, die Sulfongruppen tragen, sind besonders bevorzugt.

Die erfindungsgemäßen Monoestergemische können auch Komponenten enthalten, die sich bezüglich der Säurereste $R^1$ voneinander unterscheiden.

Bevorzugt sind auch solche erfindungsgemäßen Mischungen von Monoestern der Formeln I und II, in denen die Zusammensetzung von $R^2$ und $R^3$ dadurch definiert ist, daß

A[%] einen Wert von 4 bis 15, insbesondere von 5 bis 10,

B[%] einen Wert von 20 bis 35, insbesondere von 25 bis 31,

C[%] einen Wert von 1 bis 5, insbesondere von 2 bis 4,

D[%] einen Wert von 3 bis 20, insbesondere von 5 bis 15,

E[%] einen Wert von 5 bis 25, insbesondere von 10 bis 20,

F[%] einen Wert von 0,8 bis 5, insbesondere von 1 bis 4,

G[%] einen Wert von 1 bis 5, insbesondere von 2 bis 4, und

H[%] einen Wert von 10 bis 40, insbesondere von 20 bis 35 hat.

Bei den folgenden näheren Angaben zur Struktur der Reste $-R^2$ und $-R^3$ bedeuten R und R' gleiche oder verschiedene, vorzugsweise lineare. Alkylreste mit 7 bis 13 C-Atomen.

Die Reste $R^{2A}$ sind vorzugsweise lineare Alkyl(1) oder 2-Methyl-alkyl(1)-Reste, die sich von Alkanolen bzw. Methyl-alkanolen (Oxoalloholen) vom Strukturtyp $R-CH_2-OH$ bzw. $R-CH(CH_3)-OH$ ableiten, wobei in der Regel der Anteil der unverzweigten Alkanole überwiegt.

Die 2-Alkyl-alkyl(1)-Reste $-R^{2B}$ leiten sich vorzugsweise von verzweigten Alkanolen $HO-R^{2B}$ vom Strukturtyp $R-CH_2-CH_2-CH(CH_2OH)-R'$ ab. die sich aus Aldehyden der Formeln $R-CH_2-CHO$ und $R'-CH_2-CHO$ durch Aldolkondensation, und anschließende Wasserabspaltung und Hydrierung bilden können.

Die x-Alkyl-alkyl(y)-Reste $-R^{2C}$ leiten sich von sekundären Alkanolen $HO-R^{2C}$ vom Strukturtyp $R-CH_2-CH(OH)-CH(CH_3)-R'$ ab. Diese werden wahrscheinlich durch Aldolkondensation aus Aldehyden der Formeln $R-CH_2-CHO$ und $R'-CH_2-CHO$ und anschließende Hydrierung der Aldehydgruppe zur Methylgruppe gebildet.

Die Alkoxyalkylreste $-R^{2D}$ leiten sich von Alkanolen $HO-R^{2D}$ der Strukturtypen $R-CH_2-CH(CH_2OH)-OCH_2-R'$ und $R-CH(CH_2OH)CH_2-OCH_2-R'$ ab. Diese können sich durch Acetalisierung von $R-CH_2-CHO$ mit 2 Mol $R'-CH_2-CH_2OH$, Abspaltung von einem Mol des Alkanols unter Bildung von Vinylethern der Formel III

$$R-CH=CH-O-CH_2-CH_2-R' \quad (III),$$

und Hydroformylierung der Vinylether bilden.

Die Estergruppen tragenden Reste $-R^{2E}$ leiten sich von einem oder mehreren Alkanolen $HO-R^{2E}$ der Strukturtypen

$R-CH_2-CH(OH)-CH(CH_2-OCO-CH_2-R)-R'$,

$R-CH_2-CH(OH)-CH(CH_2-OCO-CH_2-R')-R'$,

$R-CH_2-CH(OH)-CH(COO-C_2H_4-R)-R'$

$R-CH_2-CH(OH)-CH(COO-C_2H_4-R')-R'$

$R-CH_2-CH(OCO-CH_2-R)-CH(CH_2OH)-R'$

und

$R-CH_2-CH(OCO-CH_2-R')-CH(CH_2OH)-R'$

ab. Diese können gebildet werden durch eine Cannizzaro-Reaktion (Disproportionierung) von 1 Mol eines der oben genannten Aldole mit einem Mol eines Aldehyds $R-CH_2-CHO$ oder $R'-CH_2-CHO$ und anschließende Veresterung der dabei entstandenen Carbonsäuren und Alkanole.

Die Alkandiyl-Reste $-R^{3F}$ leiten sich von Diolen $(HO)_2-R^{3F}$ der Strukturtypen $R-CH_2-CH(CH_2OH)-OH$ und $R-CH(CH_2OH)-CH_2OH$ ab. Diese können gebildet werden durch Abspaltung eines Mols Olefin aus den Alkoxygruppen der oben genannten Alkoxyalkanole.

Alkyl-alkandiyl-Reste $-R^{3G}$ leiten sich von Diolen $(HO)_2-R^{3G}$ des Struktuilyps $R-CH_2-CH(OH)-CH(CH_2OH)-R'$, die durch Hydrierung der oben genannten Aldole erhalten werden können.

Die von OH-Gruppen freien Ether entsprechen der Struktur $R-CH_2CH_2-O-CH_2-R'$. Sie werden bei der Hydrierung der Vinylether der Formel III erhalten.

Die von OH-Gruppen freien Ester können linear oder verzweigt sein.
Die linearen Ester entsprechen dem Strukturtyp $R-CH_2CH_2-OCO-CH_2R'$, und bilden sich durch Cannizzaro Disproportionierung aus Aldehyden der Formel $R-CH_2-CHO$ und $R'-CH_2-CHO$ und anschließende Veresterung der entstandenen Carbonsäuren und Alkanole.

Die verzweigten Ester entsprechen den Strukturtypen

$R-CH_2-CH_2-CH(CH_2-OCO-CH_2-R)-R'$,

$R-CH_2-CH_2-CH(CH_2-OCO-CH_2-R')-R'$,

$R-CH_2-CH_2-CH(COO-C_2H_4-R)-R'$

und

$R-CH_2-CH_2-CH(COO-C_2H_4-R')-R'$

$R-CH_2-CH(OCO-CH_2-R)-CH(CH_3)-R'$

und

$R-CH_2-CH(OCO-CH_2-R')-CH(CH_3)-R'$.

Sie können gebildet werden durch Wasserabspaltung und anschließende Hydrierung der oben genannten Estergruppen tragenden Alkanole.

Ein weiterer Gegenstand der vorliegenden Erfindung ist ein Verfahren zur Herstellung der oben beschriebenen Gemische von Monoestern der Formeln I und II durch Umsetzung innerer Anhydride zwei- oder dreibasiger Carbonsäuren mit Alkanolen mittlerer und/oder größerer Kettenlänge, das dadurch gekennzeichnet ist, daß man

A) innere Anhydride der Formel IV

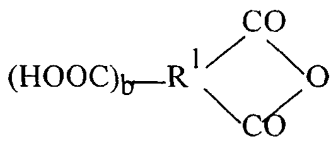

(IV)

worin b die Ziffern 0 oder 1 bedeutet und

$R^1$ ein zwei- oder dreiwertiger, gesättigter oder ein- oder zweifach ungesättigter aliphatischer oder cycloaliphatischer Kohlenwasserstoffrest mit 2 bis 6 C-Atomen oder ein zwei oder dreiwertiger aromatischer Kohlenwasserstoffrest mit 6 C-Atomen ist, der eine Sulfogruppe enthalten kann,

bei einer Temperatur von ca. 20 bis maximal 120°C, vorzugsweise von 75 bis 100°C, im Äquivalentverhältnis von 1:1 1 bis 1:5 mit einer Mischung von Alkoholen der Formel $R^2OH$, worin

$R^2$ überwiegend verzweigtes, gegebenenfalls durch Hydroxy, Alkoxy, Alkylcarbonyloxy oder Alkoxycarbonyl substituiertes Alkyl mit 9 bis 51, vorzugsweise 9 bis 45 C-Atomen bedeutet,

Alkandiolen der Formel $(HO)_2R^3$, worin

$R^3$ Alkandiyl mit 10 bis 30 C-Atomen bedeutet,

und von OH-Gruppen freien Ethern und Estern mit 18 bis 45 C-Atomen umsetzt, und,

B) falls aus einem von Sulfonsäuregruppen freien Anhydrid (IV) erfindungsgemäße Gemische hergestellt werden sollen, in denen $R^1$ eine Sulfogruppe aufweist, für den Herstellungsschritt A) ein Anhydrid IV eingesetzt wird, dessen Rest $R^1$ mindestens einfach ungesättigt ist und das gemäß Abschnitt A) erhaltene Monoester-Gemisch, gegebenenfalls nach zumindest teilweiser Neutralisation seiner freien Carboxylgruppen, in an sich bekannter Weise mit einem wasserlöslichen Sulfit, Hydrogensulfit oder Disulfit [$(S_2O_5)^{2-}$, liefert in wäßrigem Medium ebenfalls Hydrogensulfit-Anion] unter Addition des Sulfits bzw. Hydrogensulfits an die Doppelbindung der Gruppe $R^1$ unter Bildung der gewünschten Sulfonsäure umsetzt.

Die Mengen der im Herstellungsschritt A) miteinander umzusetzenden Reaktanden richtet sich nach dem Molgewicht der Anhydride der Formel IV und dem Äquivalentgewicht der Mischung der Alkohole $HOR^2$ und Diole $(HO)_2R^3$, das sich in bekannten Weise aus deren OH-Zahl ergibt.

Die Umsetzung der Di- oder Tricarbonsäureanhydride der Formel IV mit den Mischungen OH-Gruppen enthaltender Verbindungen kann mit oder ohne Lösungs- oder Verdünnungsmittel erfolgen. Sofern die Anhydride mit der Alkohol- und Diol-Mischung in einem Äquivalentverhältnis unter 1 umgesetzt werden - d.h. daß die Alkohol- und Diol-Komponenten im Überschuß vorliegen - kann dieser als Lösung- und Verdünnungsmittel für die Reaktion dienen. In der Regel wird beim Herstellungsschritt A) unter den angegebenen Reaktionsbedingungen ein ausreichender Umsatz der Reaktanten innerhalb von 2 bis 5 Stunden erreicht.

Das erfindungsgemäße Verfahren kann unter Verwendung eines einzelnen oder mehrerer verschiedener innerer Anhydride der Formel IV ausgeführt werden.

Soll im Rahmen des erfindungsgemäßen Verfahrens eine Sulfonierung durch Sulfit- oder Hydrogensulfit-Addition gemäß dem fakultativen Verfahrensschritt B) ausgeführt werden, so wird im Verfahrensschritt A) das innere Anhydrid IV mit den Alkohol- und Diol-Komponenten zweckmäßigerweise im Äquivalentverhältnis über 0,5, d.h. zwischen den Werten 1:1 und 1:2, vorzugsweise zwischen 1:1 und 1:1,2 insbesondere zwischen 1:1 und 1:1.05 ausgeführt.

Bevorzugte erfindungsgemäße Monoester-Mischungen werden erhalten wenn innere Anhydride der Formel

IV eingesetzt werden, in denen

$R^1$ ein zweiwertiger, gesättigter aliphatischer Kohlenwasserstoffrest mit 2 bis 4 C-Atomen, der durch eine Sulfonsäuregruppe substituiert sein kann, oder ein zweiwertiger, einfach ungesättigter aliphatischer Kohlenwasserstoffrest mit 2 bis 4 C-Atomen, oder ein zweiwertiger oder dreiwertiger, gesättigter cycloaliphatischer Kohlenwasserstoffrest mit 6 C-Atomen, der durch eine Sulfonsäuregruppe substituiert sein kann oder ein zwei oder dreiwertiger cycloaliphatischer Kohlenwasserstoffrest mit 6 C-Atomen, der eine oder ggf. zwei Doppelbindungen aufweist, oder ein zwei oder dreiwertiger aromatischer Kohlenwasserstoffrest mit 6 C-Atomen bedeutet.

Insbesondere eignen sich zur Herstellung bevorzugter erfindungsgemäßer Monoester-Mischungen Anhydride der Formel IV, in denen

$R^1$ Ethan-1,2-diyl, Sulfo-ethan-1,2-diyl, Ethen-1,2-diyl, Propan-1.2-diyl, Propan 1,3-diyl, Sulfopropan-1,2-diyl, Sulfopropan 1,3-dlyl. Propen-1,2-diyl, Cyclohexan-1,2-dlyl, Sulfocyclohexan-1.2-diyl. Disulfocyclohexan-1,2-diyl, Cyclohex-1-,2-,3- oder 4-en-1.2-diyl, Cyclohex-1,3-. 1,4-. 2,4-oder 2,5-dien-1,2-diyl, Cyclohexan-1,2,4-triyl. Sulfocyclohexan-1,2,4-triyl, Disulfocyclohexan-1,2,4-triyl, Cyclohex-l-,2-,3- oder 4-en-1.2,4-triyl, Cyclohex-1,3-, 1,4-, 2,4- oder 2,5-dien-1,2,4-triyl, Phenylen-1,2, 3-oder 4-Sulfophenylen-1,2 und Phenyl-1,2,4-triyl bedeutet und insbesondere solche in denen

$R^1$ für Ethen-1,2-diyl, Sulfo-ethan-1,2-diyl, Phenylen-1,2, 3- oder 4-Sulfophenylen-1,2 und Phenyl-1,2,4-triyl steht.

Als Di- oder Tricarbonsäureanhydride der Formel IV kommen beispielsweise in Betracht Bernsteinsäureanhydrid, Maleinsäureanhydrid, Glutarsäureanhydrid. Methylbernsteinsäureanhydrid, Citraconsäureanhydrid,

Hexahydrophthalsäureanhydrid (Cyclohexandicarbonsäureanhydrid), alle isomeren Dihydrophthalsäureanhydride, alle isomeren Tetrahydrophthalsäureanhydride, Phthalsäureanhydrid, und Trimellithsäureanhydrid, wovon Maleinsäureanhydrid, Phthalsäureanhydrid, und Trimellithsäureanhydrid besonders bevorzugt sind.

Vorzugsweise werden bei dem erfindungsgemäßen Verfahren Mischungen von Alkoholen $R^2OH$ und Diolen $R^3(OH)_2$ eingesetzt, die

0 bis 20 Gew.-%, vorzugsweise 4 bis 15 Gew.-% Alkanole $C_9$ - $C_{15}$

0 bis 40 Gew.-%, vorzugsweise 20 bis 35 Gew.-% 2-Alkylalkohole $C_{18}$ - $C_{30}$

0 bis 10 Gew.-%, vorzugsweise 1 bis 5 Gew.-% Sek. Alkohole $C_{18}$ - $C_{30}$

0 bis 20 Gew.-%, vorzugsweise 3 bis 20 Gew.-% Etheralkohole $C_{18}$ - $C_{30}$

0 bis 25 Gew.-%, vorzugsweise 5 bis 25 Gew.-% Esteralkohole $C_{27}$ - $C_{51}$

0 bis 7,5 Gew.-%, vorzugsweise 0,8 bis 5 Gew.-% 1,2-Diole $C_{10}$ - $C_{16}$

0 bis 8 Gew.-%, vorzugsweise 1 bis 5 Gew.-% Diole $C_{18}$ - $C_{30}$

0 bis 45 Gew.-%, vorzugsweise 10 bis 40 Gew.-% von OH-Gruppen-freie Ether $C_{18}$ - $C_{45}$ und Ester $C_{18}$ - $C_{45}$ umfassen.

Die genannten Mischungsbestandteile $R^2OH$ und $R^3(OH)_2$ können selbst Mischungen von Verbindungen verschiedener Strukturtypen darstellen, die sich aus der folgenden Tabelle 1 ergeben.

Tabelle 1

| Bezeichnung | Struktur-Typ | Anteilsbereich [Gew.-%] | Bevorzugter Anteilsbereich [Gew.-%] |
|---|---|---|---|
| Oxoalkohole $C_9$ - $C_{15}$ | $R-CH_2-OH$ bzw. $R-CH(CH_3)-OH$ | 0 bis 20 | 4 bis 15 |

Tabelle 1 (fortgesetzt)

| Bezeichnung | Struktur-Typ | Anteilsbereich [Gew.-%] | Bevorzugter Anteilsbereich [Gew.-%] |
|---|---|---|---|
| 2-Alkylalkohole $C_{18}$ - $C_{30}$ | R-$CH_2$-$CH_2$-CH($CH_2$OH)-R' | 0 bis 40 | 20 bis 35 |
| Sek. Alkohole $C_{18}$ - $C_{30}$ | R-$CH_2$-CH(OH)-CH($CH_3$)-R' | 0 bis 10 | 1 bis 5 |
| Etheralkohole $C_{18}$ - $C_{30}$ | R-$CH_2$-CH($CH_2$OH)-O$CH_2$-R' und R-CH($CH_2$OH)$CH_2$-O$CH_2$-R' | 0 bis 20 | 3 bis 20 |
| Esteralkohole $C_{27}$ - $C_{51}$ | R-$CH_2$-CH(OH)-CH ($CH_2$-OCO-$CH_2$-R)-R', R-$CH_2$-CH(OH)-CH ($CH_2$-OCO-$CH_2$-R')-R', R-$CH_2$-CH(OH)-CH (COO-$C_2H_4$-R)-R' R-$CH_2$-CH(OH)-CH (COO-$C_2H_4$-R')-R' R-$CH_2$-CH(OCO-$CH_2$-R)-CH ($CH_2$OH)-R' R-$CH_2$-CH(OCO-$CH_2$-R')-CH ($CH_2$OH)-R' | 0 bis 25 | 5 bis 25 |
| 1,2- und 1,3-Diole $C_{10}$ - $C_{16}$ | R-$CH_2$-CH($CH_2$OH)-OH und R-CH($CH_2$OH)-$CH_2$OH | 0 bis 7,5 | 0,8 bis 5 |
| Alkyl-1,3-Diole $C_{18}$ - $C_{30}$ | R-$CH_2$-CH(OH)-CH($CH_2$OH) -R' | 0 bis 8 | 1 bis 5 |
| Ether $C_{18}$ - $C_{45}$ (OH-Gruppen-frei) | R-$CH_2CH_2$-O-$CH_2$-R' | 0 bis 45 | 10 bis 40 |
| Ester $C_{18}$ - $C_{45}$ (OH-Gruppen-frei) | R-$CH_2CH_2$-O-$CH_2$-R' R-$CH_2$-$CH_2$-CH ($CH_2$-OCO-$CH_2$-R)-R', R-$CH_2$-$CH_2$-CH ($CH_2$-OCO-$CH_2$-R')-R', R-$CH_2$-$CH_2$-CH(COO-$C_2H_4$-R)-R', R-$CH_2$-$CH_2$-CH(COO-$C_2H_4$-R')-R', R-$CH_2$-CH(OCO-$CH_2$-R)-CH ($CH_3$)-R', R-$CH_2$-CH(OCO-$CH_2$-R')-CH ($CH_3$)-R', R-$CH_2$-CH(OCO-$CH_2$-R)-CH ($CH_3$)-R, R-$CH_2$-CH(OCO-$CH_2$-R')-CH ($CH_3$)-R' | | |

Die für das Verfahren einzusetzenden Mischungen der angegebenen Zusammensetzung können dadurch erhalten werden, daß man eine aus der angegebenen Substanzgruppe getroffene Auswahl miteinander mischt, wobei die Mengen so bemessen werden, daß die oben angegebenen Obergrenzen der Anteile der Mischungsbestandteile nicht überschritten werden und die Summe der %-Anteile der gemischten Komponenten 100% beträgt.

Vorzugsweise wählt man die Komponenten und ihre Anteile in der Mischung so aus, daß die Mischung eine OH-Zahl zwischen 65 und 160. insbesondere zwischen 90 und 140 mg KOH / g aufweist.

Wird beim erfindunggemäße Verfahren das innere Anhydrid IV mit dem Alkanol- und Diol-Gemisch in Äquivalenzverhältnissen von etwa 1:1 umgesetzt, so werden spezielle erfindungsgemäße Monoester-Gemische erhalten, die in der Regel weniger als 5 Gew.-%, vorzugsweise weniger als 2 Gew.%. insbesondere weniger als 0,5 Gew.-% Alkanole und Diole enthalten.

Wird das innere Anhydrid IV mit dem Alkanol- und Diol-Gemisch in Äquivalenzverhältnissen unter 1, z.B. von 1:2 oder 1:3, umgesetzt, so werden spezielle Mischungen erhalten, die höhere Anteile an Alkanolen und Diolen , z.B. ca. 37 Gew.-% bzw. 55 Gew.-%, aufweisen, die sich besonders gut zu anwendungsfertigen Lederfettungs- und -hydrophobierungs-Zubereitungen verarbeiten lassen, weil sie leicht mit weiteren Fettungs- und Hydrophobierungsmitteln und Hilfsmitteln gemischt werden können und weil auch die überschüssige Alkanol- und Diol-Komponenten eine gute fettende Wirkung zeigen.

Ein ganz besonderer Vorteil der vorliegenden Erfindung besteht darin, daß die erfindungsgemäßen Monoestergemische auf sehr einfache Weise durch Umsetzung der inneren Anhydride der Formel IV mit sogenannten Oxo-Dickölen als Alkoholkomponente erhalten werden können.

Durch den Einsatz dieser Oxo-Dicköle sind die erfindungsgemäßen Mischungen von Monoestern mehrbasiger Carbonsäuren nicht nur besonders preisgünstig zu produzieren sondern es eröffnet sich auch eine sinnvolle Verwertung der ansonsten nur als Neben- und Abfallprodukt geltenden Oxo-Dicköle. Die Verwendung der Oxo-Dicköle bei den erfindungsgemäßen Herstellungsverfahren stellt eine besonders bevorzugte Ausführungsform desselben dar und auch die so hergestellten erfindungsgemäßen Monoestergemische stellen eine besonders bevorzugte Ausführungsform der vorliegenden Erfindung dar.

Zur Ausführung der vorliegenden Erfindung eignen sich Oxo-Dicköle, die

0 bis 20 Gew.-%, vorzugsweise 4 bis 15 Gew.-% Oxoalkohole $C_9$ - $C_{15}$

0 bis 40 Gew.-%, vorzugsweise 20 bis 35 Gew.-% 2-Alkylalkohole $C_{18}$ - $C_{30}$

0 bis 10 Gew.-%, vorzugsweise 1 bis 5 Gew.-% Sek. Alkohole $C_{18}$ - $C_{30}$

0 bis 20 Gew.-%, vorzugsweise 3 bis 20 Gew.-% Etheralkohole $C_{18}$ - $C_{30}$

0 bis 25 Gew.-%, vorzugsweise 5 bis 25 Gew.-% Esteralkohole $C_{27}$ - $C_{51}$

0 bis 7,5 Gew.-%, vorzugsweise 0,8 bis 5 Gew.-% 1 2-Diole $C_{10}$- $C_{16}$

0 bis 8 Gew.-%, vorzugsweise 1 bis 5 Gew.-% Diole $C_{18}$ - $C_{30}$

0 bis 45 Gew.-%, vorzugsweise 10 bis 40 Gew.-% von OH-Gruppen-freie Ether $C_{18}$ - $C_{45}$ und Ester $C_{18}$ - $C_{45}$ umfassen,

und eine OH-Zahl im Bereich von 65 bis 160, vorzugsweise von 90 bis 140 aufweisen.

Zur Auswahl geeigneter Oxo-Dicköle ist es lediglich erforderlich, die zur Verfügung stehenden Produkte daraufhin zu untersuchen, ob sie die oben beschriebene erforderliche Zusammensetzung aufweisen.

Die qualitative Untersuchung von Oxo-Dickölen und die quantitative Bestimmung der darin enthaltenen Haupt-Bestandteile kann in an sich bekannter Weise (Vergl. z.B. EP-A-0 718 351) durch gaschromatographische Trennung mit angekoppelter massenspektrographischer Untersuchung der erhaltenen Fraktionen erfolgen.
Zweckmäßigerweise arbeitet man unter Einsatz von 25 m oder 50 m SE 54 Fused Silica Kapillaren. Abbildungen von so erhaltenen Gaschromatogrammen finden sich auf den Seiten 14 und 15 der oben genannten EP-A-0 718 351. Durch Ausmessen der Peakflächen der Hauptkomponenten und Normierung auf 100% kann die Zusammensetzung der Oxo-Dicköle mit einer für die Ausführung der vorliegenden Erfindung ausreichenden Genauigkeit ermittelt werden.

Speziell - aber keineswegs ausschließlich - geeignet zur Herstellung der erfindungsgemäßen Monoester sind die Oxodicköle der Typen "Oxoöl 911", "Oxoöl 13" und "Oxoöl 135".

Die erfindungsgemäßen Monoestergemische können, durch Überführung zumindest eines Teils der darin enthaltenen freien Carbonsäuregruppen in Metallsalze (M in Formeln I und II zumindest teilweise = Metallatom), vorzugsweise in Alkalisalze, insbesondere in Natrium- oder Kaliumsalze, d.h. nach Neutralisation oder Teilneutralisation der Carbonsäuregruppen, in eine wasserlösliche oder wasserdispergierbare Form gebracht und dann als Dispergiermittel bzw. als Emulgatoren zur stabilen Feinverteilung wasserunlöslicher Substanzen in einer wässrigen Phase eingesetzt werden, die sich durch eine sehr gute Wirksamkeit und sehr günstige Gestehungskosten auszeichnen. Die Verwendung der erfindungsgemäßen Monoestergemische als Emulgatoren ist ebenfalls ein Gegenstand der vorliegenden Erfindung.

Eine besonders vorteilhafte Verwendung, die ebenfalls ein Gegenstand dieser Erfindung ist, besteht im Einsatz der erfindungsgemäßen Monoestergemische als Hilfsmittel bei der Herstellung von Leder. Hierbei werden die erfindungsgemäßen Monoestergemische in Kombination mit Lederfettungsmitteln und/oder Lederhydrophobierungsmitteln eingesetzt.

Als fettende und/oder hydrophobierende Mittel kommen insbesondere unveresterte Oxo-dicköle in Betracht. Zusätzlich zu diesen oder an deren Stelle können jedoch auch weitere Substanzen, wie z.B. Weißöl, Paraffine, native Öle und/oder Silikone als fettende und/oder hydrophobierende Mittel eingesetzt werden.

Besonders gute Hydrophobierungseffekte werden erhalten, wenn die erfindungsgemäßen Emulgatoren mit Silikonen und den unveresterten Oxodickölen und/oder gegebenenfalls weiteren fettenden und/oder hydrophobierenden Substanzen kombiniert werden. Als Silikone, die sich für diesen Einsatz eignen, sind insbesondere bekannte Polysiloxane zu nennen, die an einer linearen oder verzweigten Siloxan-Grundkette über Brückenglieder gebundene Carboxylgruppen tragen. Diese Verbindungen entsprechen vorzugsweise der Formel V

$$[\text{Siloxan-Grundkette-}][\text{-BR(-COOH)}_p]_y, \qquad (V)$$

worin BR ein (p+1)-valentes an ein Si-Atom der Grundkette gebundenes organisches Brückenglied ist, p für eine Zahl von 1 bis 10 steht und y so gewählt wird, daß die Verbindung 0,01 bis 2,0, vorzugsweise 0,02 bis 1,5 meq/g Carboxylgruppen enthält.

Geeignete Brückenbausteine BR entsprechen beispielsweise der Formel (VI)

$$\text{-Z-(A-)}_p \qquad (VI),$$

worin A ein divalenter aliphatischer geradkettiger oder verzweigter, ein divalenter cyclischer oder bicyclischer, gesättigter oder ungesättigter oder ein divalenter aromatischer Kohlenwasserstoffrest ist, Z eine direkte Bindung ein Sauerstoffatom oder eine Gruppe der Formel $-NR^4-$, -CO-, oder —CO-O- oder einen (p+1)-valenten organischen Rest der Formel VIa

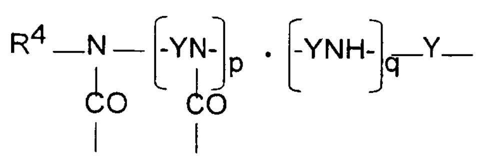

(VIa)

bedeutet, wobei p und q unabhängig voneinander für die Zahlen von 0 bis 10 stehen und die Summe p+q ebenfalls im Bereich von 0 bis 10 liegt, und $R^4$ in den genannten Baugruppen Wasserstoff oder $C_1$- bis $C_4$-Alkyl und Y gleiche oder verschiedene geradkettige oder verzweigte Alkandiyl-Reste mit 2 bis 4 C-Atomen bedeuten.

Als Beispiel für geeignete Polysiloxane mit linearer Siloxan-Grundkette sind solche der Formel VII

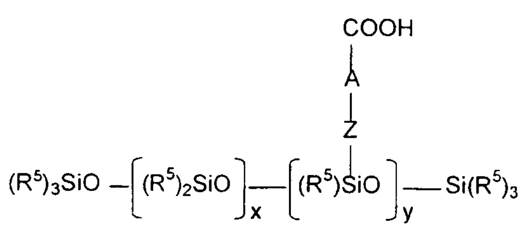

(VII)

zu nennen,
worin die Reste $R^3$ gleich oder verschieden sind und unabhängig voneinander für Wasserstoff, Hydroxyl, $C_1$- bis $C_4$-Alkyl, Phenyl, $C_1$-bis $C_4$-Alkoxy. Amino, Mono-$C_1$- bis $C_4$-alkylamino. Di-$C_1$- bis $C_4$-alkylamino, Chlor. oder Fluor stehen, wobei an den Kettenenden jeweils auch ein Rest $R^5$ für die Gruppierung -Z-A-COOH stehen kann,

A eine lineare oder verzweigte $C_5$- bis $C_{25}$-Alkylengruppe bezeichnet.

Z eine direkte Bindung, ein Sauerstoffatom oder eine Gruppe der Formel $-NR^4-$, -CO-, $-CO-NR^4-$, oder -CO-O- bedeutet, wobei $R^4$ für Wasserstoff oder $C_1$- bis $C_4$-Alkyl steht und
die Indices x und y der zugehörigen statistisch verteilten Struktureinheiten in der Summe 50 bis 500 ergeben, wobei

pro Molekül VII im statistischen Mittel 1 bis 50, vorzugsweise 2 bis 20, insbesondere 2,5 bis 15 Carboxylgruppen vorhanden sind.

Besonders gut geeignet zur Kombination mit den erfindungsgemäßen Monoestern sind Siloxane der Formel VII, in denen die Summe x+y im Bereich von 100 bis 300, insbesondere von 120 bis 200, und das Verhältnis x:y im Bereich von 99:1 bis 9:1 liegt, sowie solche, bei denen $R^5$ für $C_1$- bis $C_3$-Alkyl, insbesondere für Methyl, steht. Siloxane der Formel VII sind bekannt aus der EP-B-0 745 141.

Als Beispiele für Siloxane mit linearer oder verzweigter Grundkette seien die aus der WO-98/21369 bekannten Verbindungen der Formel VIII

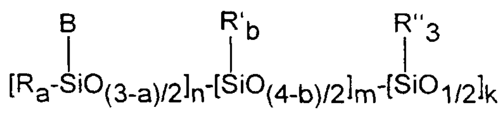

(VIII)

zu nennen, worin R.R' und R" unabhängig voneinander $C_1$- bis $C_6$-Alkyl oder Phenyl oder ein Polysiloxanrest der Formel VIIIa

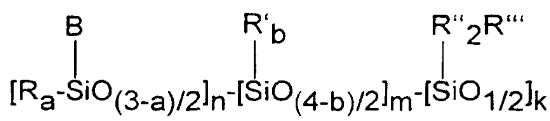

(VIIIa)

ist, wobei in der Formel VIII R' und R" unabhängig voneinander $C_1$- bis $C_6$-Alkyl oder Phenyl und R und R''' unabhängig voneinander $C_1$- bis $C_6$-Alkyl, $C_1$- bis $C_6$-Alkoxy, OH oder Phenyl bedeutet, und

$$0 \le a \le 2,\ 1 \le b \le 3,\ 1 \le n \le 60,\ 20 \le m \le 800, \text{ und } 0 \le k \le (2-b)m + [(1-a)n + 2]$$

ist,
und B einen Rest der Formel IX

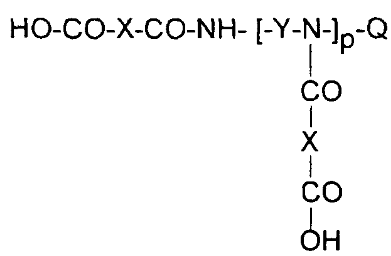

(IX)

darstellt, worin p eine Zahl von 0 bis 10 bedeutet, Y und Q unabhängig voneinander Alkandiyl kurzer oder mittlerer Kettenlänge und X ein divalenter aliphatischer, gesättigter oder ungesättigter, geradkettiger oder verzweigter, ein divalenter cyclischer oder bicyclischer, gesättigter oder ungesättigter oder ein divalenter aromatischer Kohlenwasserstoffrest ist,
wobei wahlweise ein Teil der Substituenten der Formel IX durch Reste der Formel X oder XI

$$H_2N\text{-} [\text{-}Y\text{-}NH\text{-}]_p\text{-}Q\text{-}$$

(X)

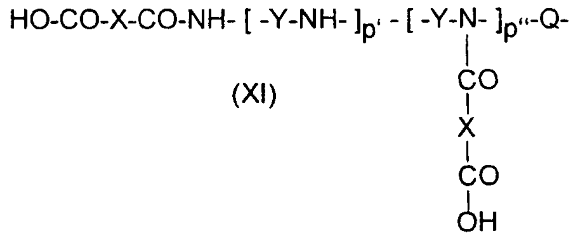

(XI)

ersetzt sein kann,
worin X,Y und Q und p die oben angegebenen Bedeutungen haben und
p' + p" = p ist. Die Polysiloxane der Formeln VIII und VIIIa haben einen Carboxylgruppen-Gehalt von 0,02 bis 1,0 meq/g und eine Molmasse im Bereich von 2 x $10^3$ bis 60 x $10^3$ g/Mol.

Für den bevorzugten Einsatz für Lederhilfsmittel werden zweckmäßigerweise wäßrige Zubereitungen eingesetzt, die neben den erfindungsgemäßen Emulgatoren die zur Fettung und/oder Hydrophobierung erforderlichen Substanzen als Emulsion in Wasser enthalten. Im Prinzip werden zur Emulgierung der Fettungs- und Hydrophobierungsmittel neben den erfindungsgemäßen Emulgatoren keine weiteren amphiphilen Komponenten benötigt; sie können aber zugesetzt werden, wenn spezielle Effekte gewünscht werden. Als fettende und/oder hydrophobierende Komponenten kommen für diese Zubereitungen die oben genannten Substanzen, insbesondere unveresterte Oxodicköle in Betracht. Neben oder anstelle von unveresterten Oxodickölen können jedoch auch weitere Substanzen, wie z.B. Weißöl, Paraffine, native Öle und/oder Silikone in die Zubereitungen eingearbeitet werden, wobei Zubereitungen, die einen Anteil von Silikonen, vorzugsweise der oben angegebenen, Siloxane der Formel V, insbesondere solcher der Formeln VII oder VIII, enthalten, besonders hohe Hydrophobierungseffekte ergeben. Zweckmäßigerweise enthalten derartige Zubereitungen, 1 bis 20, vorzugsweise 2 bis 10, insbesondere 2,5 bis 8 Gew.-% des Silikons, insbesondere des Siloxans der Formel V.

Die besagten wäßrigen Zubereitungen sind ebenfalls ein Gegenstand der vorliegenden Erfindung.

Sie enthalten in der Regel neben Wasser, bezogen auf den nicht flüchtigen Anteil

10 bis 40 Gew.-% der erfindungsgemäßen Monoester und/oder deren Salze,

0 bis 20 Gew.-% weitere Emulgiermittel

0 bis 90 Gew.-% nicht verestertes Oxodicköl

0 bis 90 Gew.-% weitere hydrophobierende Substanzen der oben genannten Klassen,

0 bis 10 Gew.-% Hilfsmittel, wie Schaumdämpfungsmittel, Frostschutzmittel. Bakterizide, Fungizide. Metallkomplexbildner, Lagerstabilisatoren. Verdünnungshilfsmittel und dergleichen.

Der Feststoffgehalt der wäßrigen Zubereitungen liegt zweckmäßigerweise bei 20 bis 60 Gew.-%. kann aber, wenn es gewünscht wird, niedriger oder höher eingestellt und damit besonderen Anwendern. Anwendungsbereichen und Anlagenerfordernissen angepaßt werden.

Selbstverständlich ist es auch möglich, wasserfreie Zubereitungen der oben angegebenen Zusammensetzung herzustellen und bei Bedarf einzusetzen. Auch diese wasserfreien Zubereitungen sind daher Gegenstand dieser Erfindung.

Die erfindungsgemäßen wäßrigen Zubereitungen stellen relativ niederviskose Flüssigkeiten dar, weisen vergleichsweise niedrige Emulgatorkonzentrationen auf, haben eine sehr gute Lagerstabilität, sind sehr unempfindlich gegen Wasserhärte und Nachgerbstoffe und lassen sich ohne Schwierigkeiten auf die für die Lederbehandlung erforderliche Anwendungskonzentration verdünnen.

Die damit behandelten Leder haben keine schmierige Oberfläche, einen angenehmen Griff, sind gleichmäßig gefärbt und wasserundurchlässig. Die erhaltenen Effekte zeigen eine sehr gute Beständigkeit gegen Wasser, wäßrige Tensidlösungen und chemische Reinigungsmittel.

Auch der Einsatz der erfindungsgemäßen Monoester als Hilfsmittel in Lederbehandlungsverfahren ist ein Gegenstand der vorliegenden Erfindung. Für diesen Einsatz werden die Monoester zweckmäßigerweise in Form der oben beschriebenen Zubereitungen, vorzugsweise der wäßrigen Zubereitungen, verwendet. In der Regel werden den Lederbehandlungsflotten, bezogen auf das Falzgewicht des Leders (wet blue) 0,5 bis 8, vorzugsweise 1.5 bis 5 Gew.-

% der in der erfindungsgemäßen Zubereitungen enthaltenen nichtflüchtigen Anteile zugesetzt. Im Übrigen erfolgt die Lederbehandlung, in bekannter Weise.

Die folgenden Ausführungsbeispiele dienen der Veranschaulichung der Erfindung. Die Zusammensetzung des darin eingesetzten Typs von Oxodicköl ist der folgenden Tabelle 2 zu entnehmen:

Tabelle 2

| Oxodicköl-Type | "Oxoöl 135" |
|---|---|
| Komponenten | Anteile in Gew.-% |
| Oxoalkohole $C_9$ - $C_{15}$ | 6,1 |
| 2-Alkylalkohole $C_{18}$ - $C_{30}$ | 27,9 |
| Sek. Alkohole $C_{18}$ - $C_{30}$ | 4,1 |
| Etheralkohole $C_{18}$ - $C_{30}$ | 6,7 |
| Esteralkohole $C_{27}$ - $C_{45}$ | ca. 17,4 |
| 1,2-Diole $C_{10}$ - $C_{16}$ | 1,0 |
| Diole $C_{18}$ - $C_{30}$ | 2,3 |
| Ether $C_{18}$ - $C_{45}$ (OH-Gruppen-frei) | 5,4 |
| Ester $C_{18}$ - $C_{45}$(OH-Gruppen-frei) | ca. 29,1 |

Die qualitative Untersuchung der Oxoöle 911 und 135 und die quantitative Bestimmung der in Tabelle 2 angegebenen Anteilswerte der Bestandteile erfolgte in an sich bekannter Weise durch gaschromatographische Trennung und angekoppelter massenspektrographischer Untersuchung der erhaltenen Fraktionen. Die Trennung wurde bei Oxoöl 911 unter Einsatz einer 50 m SE 54 Fused Silica Kapillare ausgeführt. Für die Trennung des Oxoöls 135 wurde eine entsprechende 25 m Kapillare verwendet. Die in der Tabelle angegebenen Hauptkomponenten wurden durch Normierung der GC-Peakflächen auf 100% ermittelt.

Das in den Beispielen eingesetzte Oxoöl 135 ist außerdem durch eine OH-Zahl von 117 charakterisiert. Die Bestimmung der OH-Zahl erfolgte in Anlehnung an die Norm DIN-53240 vom Dezember 1971 bzw. DIN-53240 Teil II vom Dezember 1993.

Die Prüfung der Leder auf Wasserdurchlässigkeit erfolgte mit dem Bally-Penetrometer gemäß IUP 10 der Internationalen Union der Leder-Chemiker-Verbände.(Vergl. "Das Leder", Bd. 12, Seiten 26-40 (1961))

**Beispiel 1.**

A) Herstellung eines erfindungsgemäßen Monoesters.

In einem 1000 ml Dreihals-Glaskolben, ausgerüstet mit Rührer, Thermometer, Rückflußkühler und Feuchtigkeitsverschluß werden 470 g (0,9 Mol nach OH-Zahl) Oxodicköl Typ 135 vorgelegt und im Ölbad auf 100°C erwärmt. Unter Rühren werden dann 133,3 g (0,9 Mol) Phthalsäureanhydrid eingetragen und die Mischung 7 Stunden bei 100°C gerührt.

Der so erhaltene Monoester wird auf 40°C abgekühlt; der Umsatz ist praktisch quantitativ.

B) Herstellung einer erfindungsgemäßen Zubereitung.

200 g des hergestellten Monoesters werden bei 40°C unter Rühren durch vorsichtigen Zusatz von 25 gew.-%iger Natronlauge auf einen pH-Wert von 8 eingestellt. Danach werden 340 g Oxodicköl Typ 135, 225 g Weißöl und 1200 g Wasser eingerührt und die Mischung in üblicher Weise, z.B. mittels eines Spalthomogenisators, in eine Emulsion überführt.

Die dünnflüssige Emulsion enthält 10 Gew.-% des erfindungsgemäßen Emulgators. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.

In analoger Weise können auch Oxodicköle der Typen 911 und 13 zu Halbestern umgesetzt und in Zubereitungen überführt werden.

**Beispiel 2.**

A) Herstellung eines erfindungsgemäßen Monoesters.

In einem 500 ml Dreihals-Glaskolben, ausgerüstet mit Rührer, Thermometer. Rückflußkühler und Feuchtigkeitsverschluß werden 183 g (0,3 Mol nach OH-Zahl) Oxodicköl Typ 135 vorgelegt und im Ölbad auf 100°C erwärmt. Unter Rühren werden dann 29,4 g (0,3 Mol) Maleinsäureanhydrid eingetragen und die Mischung 5 Stunden bei 100°C gerührt.
Der so erhaltene Monoester wird auf 40°C abgekühlt; der Umsatz ist praktisch quantitativ.

B) Herstellung einer erfindungsgemäßen Zubereitung.

200 g des hergestellten Monoesters werden wie in Beispiel 1 beschrieben, in eine Emulsion überführt.
Die dünnflüssige Emulsion enthält 10 Gew.-% des erfindungsgemäßen Emulgators. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.
In analoger Weise können auch Oxodicköle der Typen 911 und 13 zu Halbestern umgesetzt und in Zubereitungen überführt werden.

**Beispiel 3.**

A) Herstellung eines erfindungsgemäßen Monoesters.

In einem 1000 ml Dreihals-Glaskolben, ausgerüstet mit Rührer, Thermometer. Rückflußkühler und Feuchtigkeitsverschluß werden 549 g (0.9 Mol nach OH-Zahl) Oxodicköl Typ 135 vorgelegt und im Ölbad auf 100°C erwärmt. Unter Rühren werden dann 29,4 g (0,3 Mol) Maleinsäureanhydrid eingetragen und die Mischung 3 Stunden bei 100°C gerührt.
Der so erhaltene Monoester wird auf 40°C abgekühlt; der Umsatz ist praktisch quantitativ.

B) Herstellung einer erfindungsgemäßen Zubereitung.

Das gemäß Abschnitt A erhaltene Gemisch aus erfindungsgemäßem Monoester und überschüssigem Oxodicköl 135 wird bei 40°C unter Rühren durch vorsichtigen Zusatz von 25 gew.-%iger Natronlauge auf einen pH-Wert von 8 eingestellt. Danach werden 225 g Weißöl und 1300 g Wasser eingerührt und die Mischung in üblicher Weise, z.B. mittels eines Spalthomogenisators, in eine Emulsion überführt.
Die dünnflüssige Emulsion enthält 10 Gew.-% des erfindungsgemäßen Emulgators. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH. In analoger Weise können auch Oxodicköle der Typen 911 und 13 umgesetzt und in Zubereitungen überführt werden.

**Beispiel 4.**

A) Herstellung eines erfindungsgemäßen, eine Sulfonsäuregruppe enthaltenden Monoesters.

In einem 1000 ml Dreihals-Glaskolben, ausgerüstet mit Rührer, Thermometer, Rückflußkühler und Feuchtigkeitsverschluß werden 695 g (1,4 Mol nach OH-Zahl) Oxodicköl Typ 135 vorgelegt und im Ölbad auf 100°C erwärmt. Unter Rühren werden dann 132,2 g (1,4 Mol) Maleinsäureanhydrid eingetragen und die Mischung 5 Stunden bei 100°C gerührt.
Der so erhaltene Monocstcr wird auf 40°C abgekühlt, in 965 g Wasser eingerührt, durch Zusatz von 61,2 g (0,77 Mol) 50 gew.-%-iger Natronlauge teilneutralisiert und die erhaltene Mischung auf 80°C erwärmt. Dann werden unter Rühren 133,1 1 g Natriumdisulfit zugefügt. Und der Ansatz bei 80°C 6 Stunden weitergerührt.

B) Herstellung einer erfindungsgemäßen Zubereitung.

400 g der in Abschnitt A hergestellten 50 gew.-%igen Mischung des Monoester/Sulfit-Addukts mit Wasser werden bei 40°C unter Rühren durch vorsichtigen Zusatz von 25 gew.-%iger Natronlauge auf einen pH-Wert von 8 eingestellt. Danach werden 340 g Oxodicköl Typ 135, 225 g Weißöl und 1000 g Wasser eingerührt und die Mischung in üblicher Weise, z.B. mittels eines Spalthomogenisators, in eine Emulsion überführt.

Die dünnflüssige Emulsion enthält 10 Gew.-% des erfindungsgemäßen Emulgators. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.
In analoger Weise können auch Oxodicköle der Typen 911 und 13 umgesetzt und in Zubereitungen überführt werden.

**Beispiel 5.**

A) Herstellung eines erfindungsgemäßen, eine Sulfonsäuregruppe enthaltenden Monoesters.

Der in Abschnitt A des Beispiels 4 beschriebene Ansatz wird wiederholt.

B) Herstellung einer erfindungsgemäßen Zubereitung.

400 g der in Abschnitt A hergestellten 50 gew.-%igen Mischung des Monoester/Sulfit-Addukts mit Wasser werden bei 40°C unter Rühren durch vorsichtigen Zusatz von 25 gew.-%iger Natronlauge auf einen pH-Wert von 8 eingestellt. Danach werden 340 g Oxodicköl Typ 135, 225 g Weißöl. 80 g eines Silicons der unten angegebenen Formel XII und 950 g Wasser eingerührt und die Mischung in üblicher Weise, z.B. mittels eines Spalthomogenisators, in eine Emulsion überführt.
Die dünnflüssige Emulsion enthält 10 Gew.-% des erfindungsgemäßen Emulgators. Sie hat eine sehr gute Lagerbeständigkeit (Stabilität bei 23°C, 40°C und 50°C mehr als 60 Tage) und läßt sich sehr leicht mit Wasser auf Anwendungskonzentration verdünnen. Sie ist beständig gegenüber Hartwasser bis mindestens 40°dH.
In analoger Weise können auch Oxodicköle der Typen 911 und 13 umgesetzt und in Zubereitungen überführt werden.
Das in diesem Beispiel eingesetzte Silicon hat die folgende Formel XII:

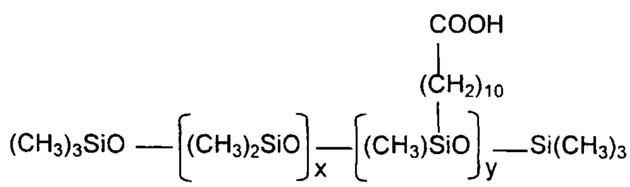

(XII)

worin die Summe von x und y ca. 140 bis 150 beträgt und y einen Wert von ca. 3 hat.

**Eigenschaften erfindungsgemäßer Zubereitungen**

Die gemäß den Beispielen 1 bis 4 erhaltenen Zubereitungen haben, wie oben angegeben, eine sehr gute Lagerbeständigkeit und lassen sich ohne Schwierigkeiten mit Wasser auf Anwendungskonzentration verdünnen.
Selbst eine gemäß Beispiel 4 hergestellte erfindungsgemäße Zubereitung, die nur 5 Gew.-% des erfindungsgemäßen Emulgators enthielt, weist bei 23°C, 40°C und 60°C eine Lagerstabilität von über 60 Tagen auf.
Zum Vergleich wurde eine Zubereitung gemäß Beispiel 4, Abschnitt B. hergestellt, die anstelle des erfindungsgemäßen Emulgators 10 Gew.% N-Oleoylsarkosin, eines handelsüblichen, sehr gut wirksamen, und daher trotz seines relativ hohen Preises im Hydrophobiermittel-Bereich verbreiteten Emulgators, enthielt. Diese Zubereitung zeigte eine erheblich geringere Lagerstabilität: Bei einer Lagerung bei 23°C trat bereits nach 6 Tagen eine wäßrige Trennung auf (20% der Emulsion sind relativ klar).
Erst eine Zubereitung mit 15 Gew.-% dieses handelsüblichen Emulgators blieb bei einer 23°C-Lagerung mehr als 14 Tage stabil.
Schon allein der Mengenvergleich der zur Stabilisierung der Zubereitungen benötigten Emulgatoren zeigt, daß erst die etwa dreifache Menge des Sarkosin-Emulgators annähernd die Wirksamkeit des erfindungsgemäßen Emulgatorsystems erreicht.
Zieht man darüberhinaus die Wirkungs/Kosten-Relation in Betracht, dann ergibt sich eine mindestens zehnfache Überlegenheit des erfindungsgemäßen Emulgators gegenüber dem guten, handelsüblichen Produkt.

**Anwendungstechnische Beispiele.**

**Beispiel 6.**

Chromgegerbtes Rindleder (wetblue) der Falzstärke 1,8 - 2,0 mm, das auf einen pH-Wert von 5,0 entsäuert

worden ist, wurde, bezogen auf Falzgewicht,

| | |
|---|---|
| 30 min. mit | 2 Gew.-% eines handelsüblichen Polymergerbstoffs, danach |
| 60 min. mit | 3 Gew.-% handelsüblichem Mimosaextrak. |
| | 3 Gew.-% eines handelsüblichen Harzgerbstoffs auf Basis von Melaminkondensationsprodukten und 1 Gem.-% eines handelsüblichen Nachgerbstoffs auf Basis von Phenolkondensationsprodukten, und danach |
| 60 min. mit | 2 Gew.-%.eines handelsüblichen Lederfarbstoffs |

im Gerbfaß gewalkt.

Anschließend wurde, bezogen auf Falzgewicht,
90 min. mit 10 Gew.-% einer Zubereitung, hergestellt gemäß Beispiel 1 gewalkt und die Flotte mit Ameisensäure auf einen pH-Wert von ca. 3.6 bis 3.8 abgesäuert und danach gewaschen. Abschließend erfolgte für die Dauer von 90 min. eine Mineralsalzfixierung mit 3 Gew.-% eines handelsüblichen Chromgerbstoffs im Gerbfaß.

Dann wurde das Leder gewaschen, mechanisch ausgereckt und getrocknet.

Das erhaltene Leder war weich, geschmeidig, mit angenehmem Griff und gleichmäßiger Färbung.

Die Prüfung mittels Bally-Penetrometer bei 15 % Stauchung erbrachte folgende Ergebnisse:

| | |
|---|---|
| Wasserdurchtritt | nach 60 min., |
| dynamische Wasseraufnahme | 30 Gew.-% nach 6 Stunden. |

**Beispiel 7**

Man arbeitet genau wie im Beispiel 6 beschrieben, jedoch wird anstelle der Zubereitung aus Beispiel 1 die gleiche Menge der Zubereitung gemäß Beispiel 5 (Zubereitung mit Silikon-Zusatz) eingesetzt.

Die Prüfung mittels Bally-Penetrometer bei 15 % Stauchung erbrachte folgende Ergebnisse:
Kein Wasserdurchtritt nach 24 Stunden,

| | |
|---|---|
| dynamische Wasseraufnahme | 22 Gew.-% nach 24 Stunden. |

**Patentansprüche**

**1.** Gemische von Monoestern zwei- oder dreibasiger Carbonsäuren der Formeln I und II,

$$(MOOC)_a\text{-}R^1\text{-CO-OR}^2 \qquad (I)$$

$$[(MOOC)_a\text{-}R^1\text{-CO-O}]_2R^3 \qquad (II)$$

worin a die Ziffern 1 oder 2 bedeutet, M für Wasserstoff oder ein Metalläquivalent steht und

$R^1$ ein zwei- oder dreiwertiger, gesättigter oder ein- oder zweifach ungesättigter aliphatischer oder cycloaliphatischer Kohlenwasserstoffrest mit 2 bis 6 C-Atomen, der durch eine Sulfonsäuregruppe substituiert sein kann, oder ein zwei oder dreiwertiger aromatischer Kohlenwasserstoffrest mit 6 C-Atomen,

$R^2$ überwiegend verzweigtes, gegebenenfalls durch Hydroxy, Alkoxy, Alkylcarbonyloxy oder Alkoxycarbonyl substituiertes Alkyl mit 9 bis 51 C-Atomen und

$R^3$ Alkandiyl mit 10 bis 30 C-Atomen bedeutet,

mit von OH-Gruppen freien Ethern und Estern mit 18 bis 45 C-Atomen und gegebenenfalls Alkanolen der Formel $R^2OH$ und Alkandiolen der Formel $R^3(OH)_2$, wobei, - bezogen auf die Summe SUG der Gewichte der in der Mischung enthaltenen von OH-Gruppen freien Ether, von OH-Gruppen freien Ester, Alkanole, Alkandiole und $-OR^2$- und $(-O)_2R^3$-Gruppen, - der Anteil der $-OR^2$-Gruppen bis zu 85 %, der Anteil der $(-O)_2R^3$-Gruppen bis zu 16 %, und der Anteil der von OH-Gruppen freien Ether und Ester bis zu 45 % beträgt.

**2.** Gemische gemäß Anspruch 1, **dadurch gekennzeichnet, dass**

$R^1$ ein zweiwertiger, gesättigter aliphatischer Kohlenwasserstoffrest mit 2 bis 4 C-Atomen, der durch eine Sulfonsäuregruppe substituiert sein kann, oder ein zweiwertiger, einfach ungesättigter aliphatischer Kohlenwasserstoffrest mit 2 bis 4 C-Atomen,
oder ein zweiwertiger oder dreiwertiger, gesättigter cycloaliphatischer Kohlenwasserstoffrest mit 6 C-Atomen, der durch eine Sulfonsäuregruppe substituiert sein kann
oder ein zwei oder dreiwertiger cycloaliphatischer Kohlenwasserstoffrest mit 6 C-Atomen, der eine oder ggf. zwei Doppelbindungen aufweist,
oder ein zwei- oder dreiwertiger aromatischer Kohlenwasserstoffrest mit 6 C-Atomen bedeutet

$R^2$ die Reste $R^{2A}$, $R^{2B}$, $R^{2C}$, $R^{2D}$ und $R^{2E}$ umfasst, wobei

$R^{2A}$ Alkyl(1)- und Alkyl(2)-Reste mit 9 bis 15 C-Atomen, mit einem mittleren Molgewicht von MA

$R^{2B}$ 2-Alkyl-alkyl(1)-Reste mit 18 bis 30 C-Atomen, mit einem mittleren Molgewicht von MB

$R^{2C}$ x-Alkyl-alkyl(y)-Reste mit 18 bis 30 C-Atomen, mit einem mittleren Molgewicht von MC,
wobei x die Position der Alkyl-Seitenkette an der Alkyl-Hauptkette und y die Position der Bindungsstelle der Alkyl-Hauptkette an das Sauerstoffatom bedeutet,

$R^{2D}$ Alkoxyalkyl(1)-Reste mit 18 bis 30 C-Atomen, mit einem mittleren Molgewicht von MD

$R^{2E}$ x-Alkylcarbonyloxy-alkyl(y)-Reste und/oder x-Alkoxycarbonyl-alkyl(y)-Reste mit 27 bis 51 C-Atomen mit einem mittleren Molgewicht von ME bedeutet,
wobei x die Position des Alkylcarbonyloxy- bzw. Alkoxycarbonyl-Substituenten an der Alkyl-Hauptkette und y die Position der Bindungsstelle der Alkyl-Hauptkette an das Sauerstoffatom bedeutet,

$R^3$ die Reste $R^{3F}$ und $R^{3G}$ umfasst, wobei

$R^{3F}$ Alkandiyl(1,2) und/oder 2-Alkyl-alkandiyl(1,3) mit 10 bis 16 C-Atomen, mit einem mittleren Molgewicht von MF

$R^{3G}$ Alkyl-alkandiyl(1,3) mit 18 bis 30 C-Atomen ist, mit einem mittleren Molgewicht von MG bedeutet,

mit von OH-Gruppen freien Ethern und Estern mit 18 bis 45 C-Atomen, und gegebenenfalls Alkanolen der Formel $R^2OH$ und Alkandiolen der Formel $R^3(OH)_2$,
und dass, - bezogen auf die Summe SUG der Gewichte der in der Mischung enthaltenen von OH-Gruppen freien Ether und Ester, Alkanole, Alkandiole und $-OR^2$- und $(-O)_2R^3$-Gruppen, -

die Gruppen $-OR^{2A}$ einen Anteil von A[%] = 0 bis 20 Gew.-%,
die Gruppen $-OR^{2B}$ einen Anteil von B[%] = 0 bis 40 Gew.-%,
die Gruppen $-OR^{2C}$ einen Anteil von C[%] = 0 bis 10 Gew.-%,
die Gruppen $-OR^{2D}$ einen Anteil von D[%] = 0 bis 20 Gew.-%,
die Gruppen $-OR^{2E}$ einen Anteil von E[%] = 0 bis 25 Gew.-%,
die Gruppen $(-O)_2R^{3F}$- einen Anteil von F[%] = 0 bis 7,5 Gew.-%,
die Gruppen $(-O)_2R^{3G}$- einen Anteil von G[%] = 0 bis 8 Gew.-%,

und die von OH-Gruppen freien Ether und Ester einen Anteil von H[%] = 0 bis 45 Gew.-% haben.

**3.** Gemische gemäß den Ansprüchen 1 und 2, **dadurch gekennzeichnet, dass** die Anteile der in den Baugruppen $-OR^2$- und $-OR^3$-enthaltenen Strukturen $-OR^{2A}$, $-OR^{2B}$, $-OR^{2C}$, $-OR^{2D}$, $-OR^{2E}$, $-OR^{3F}O$- und $-OR^{3G}O$- und der Anteil der Ether und Ester im Rahmen der oben angegebenen Grenzen so gewählt werden, daß die durch die Gleichung (GL1)

$$OHZ=561 \bullet \left( \frac{A[\%]}{MA} + \frac{B[\%]}{MB} + \frac{C[\%]}{MC} + \frac{D[\%]}{MD} + \frac{E[\%]}{ME} \right) + 112 \bullet \left( \frac{F[\%]}{MF} + \frac{G[\%]}{MG} \right) \quad (GL1)$$

- worin A[%], B[%], C[%], D[%] E[%], F[%] und G[%] die oben genannten prozentualen Anteile der in den Baugruppen -$OR^2$ und -$OR^3$ enthaltenen Strukturen, bezogen auf das Summengewicht SUG, und MA, MB, MC, MD ME, MF und MG die Molekulargewichte der besagten Strukturen sind, - definierte Größe OHZ im Bereich von 65 bis 160 liegt.

**4.** Verfahren zur Herstellung der Gemische von Monoestern der Formeln I und II des Anspruchs 1 durch Umsetzung innerer Anhydride zwei- oder dreibasiger Carbonsäuren mit Alkanolen mittlerer und/oder größerer Kettenlänge, **dadurch gekennzeichnet, dass** man

A) innere Anhydride der Formel IV

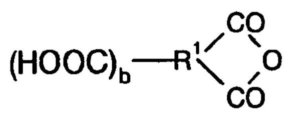 (IV)

worin b die Ziffern 0 oder 1 bedeutet und

$R^1$ ein zwei- oder dreiwertiger, gesättigter oder ein- oder zweifach ungesättigter aliphatischer oder cycloaliphatischer Kohlenwasserstoffrest mit 2 bis 6 C-Atomen oder ein zwei oder dreiwertiger aromatischer Kohlenwasserstoffrest mit 6 C-Atomen ist, der eine Sulfogruppe tragen kann,

bei einer Temperatur von ca. 20 bis maximal 120°C im Äquivalentverhältnis von 1:1 bis 1:5 mit einer Mischung von
Alkoholen der Formel $R^2OH$, worin

$R^2$ überwiegend verzweigtes, gegebenenfalls durch Hydroxy, Alkoxy, Alkylcarbonyloxy oder Alkoxycarbonyl substituiertes Alkyl mit 9 bis 51 C-Atomen bedeutet,

Alkandiolen der Formel $(HO)_2R^3$, worin

$R^3$ Alkandiyl mit 10 bis 30 C-Atomen bedeutet,

und von OH-Gruppen freien Ethern und Estern mit 18 bis 45 C-Atomen umsetzt, und,

B) falls aus einem von Sulfonsäuregruppen freien Anhydrid (IV) erfindungsgemäße Gemische hergestellt werden sollen, in denen $R^1$ eine Sulfogruppe aufweist, für den Herstellungsschritt A) ein Anhydrid IV eingesetzt wird, dessen Rest $R^1$ mindestens einfach ungesättigt ist und dann das gemäß Abschnitt A) erhaltene Monoester-Gemisch, gegebenenfalls nach zumindest teilweiser Neutralisation seiner freien Carboxylgruppen, in an sich bekannter Weise mit einem wasserlöslichen Sulfit, Hydrogensulfit oder Disulfit unter Addition des Sulfits bzw. Hydrogensulfits an die Doppelbindung der Gruppe $R^1$ unter Bildung der gewünschten Sulfonsäure umsetzt.

**5.** Verfahren gemäß Anspruch 4, **dadurch gekennzeichnet, dass** Mischungen von Alkoholen $R^2OH$ und Diolen $R^3(OH)_2$ eingesetzt, die

0 bis 20 Gew.-% Alkanole $C_9$ - $C_{15}$
0 bis 40 Gew.-% 2-Alkylalkohole $C_{18}$ - $C_{30}$
0 bis 10 Gew.-% Sek. Alkohole $C_{18}$ - $C_{30}$
0 bis 20 Gew.-% Etheralkohole $C_{18}$ - $C_{30}$

0 bis 25 Gew.-% Esteralkohole $C_{27}$ $C_{51}$
0 bis 7,5 Gew.-% 1,2-Diole $C_{10}$ - $C_{16}$
0 bis 8 Gew.-% Diole $C_{18}$ - $C_{30}$
0 bis 45 Gew.-% von OH-Gruppen-freie Ether $C_{18}$ - $C_{45}$ und Ester $C_{18}$ - $C_{45}$ umfassen.

6. Verfahren gemäß den Ansprüchen 4 und 5, **dadurch gekennzeichnet, dass** die inneren Anhydride der Formel IV mit Oxodickölen als Alkoholkomponente umgesetzt werden.

7. Verwendung der Monoestergemische des Anspruchs 1 nach Neutralisation oder Teilneutralisation der Carbonsäuregruppen, als Emulgatoren.

8. Verwendung der Monoestergemische des Anspruchs 1 als Hilfsmittel bei der Lederherstellung.

9. Verwendung gemäß Anspruch 7, **dadurch gekennzeichnet, dass** die Monoestergemische des Anspruchs 1 in Kombination mit Lederfettungsmitte1n' und/oder Lederhydrophobiermitteln eingesetzt werden.

10. Zubereitungen die neben einem wirksamen Anteil der Monoestergemische des Anspruchs 1 zur Fettung und/oder Hydrophobierung von Leder erforderliche fettende und/oder hydrophobierende Substanzen enthalten.

## Claims

1. Mixtures of monoesters of di- or tribasic carboxylic acids of the formulae I and II

$$(MOOC)_a\text{-}R^1\text{-}CO\text{-}OR^2 \quad (I)$$

$$[(MOOC)_a\text{-}R^1\text{-}CO\text{-}O]_2R^3 \quad (II)$$

where a is 1 or 2, M is hydrogen or one metal equivalent,

$R^1$ is a di- or trivalent saturated or mono- or diunsaturated aliphatic or cycloaliphatic hydrocarbon radical of 2 to 6 carbon atoms with or without sulfonic acid group substitution or is a di- or trivalent aromatic hydrocarbon radical of 6 carbon atoms,

$R^2$ is predominantly branched unsubstituted or hydroxyl-, alkoxy-, alkylcarbonyloxy- or alkoxycarbonyl-substituted alkyl of 9 to 51 carbon atoms, and

$R^3$ is alkanediyl of 10 to 30 carbon atoms,

with OH-free ethers and esters of 18 to 45 carbon atoms and optionally alkanols of the formula $R^2OH$ and alkanediols of the formula $R^3(OH)_2$, the fraction of $-OR^2$ groups being up to 85%, the fraction of $(-O)_2R^3$ groups being up to 16% and the fraction of OH-free ethers and esters being up to 45%, based on the sum total SUG of the weights of the OH-free ethers, OH-free esters, alkanols, alkanediols and $-OR^2$- and $(-O)_2R^3$groups present in the mixture.

2. Mixtures as claimed in claim 1, wherein

$R^1$ is a divalent saturated aliphatic hydrocarbon radical of 2 to 4 carbon atoms with or without sulfonic acid group substitution
or is a divalent monounsaturated aliphatic hydrocarbon radical of 2 to 4 carbon atoms,
or .is a divalent or trivalent saturated cycloaliphatic hydrocarbon radical of 6 carbon atoms with or without sulfonic acid group substitution
or is a di- or trivalent cycloaliphatic hydrocarbon radical of 6 carbon atoms having one or if appropriate two double bonds
or is a di- or trivalent aromatic hydrocarbon radical of 6 carbon atoms,

$R^2$ comprises the radicals $R^{2A}$, $R^{2B}$, $R^{2C}$, $R^{2D}$ and $R^{2E}$, where

$R^{2A}$ denotes 1-alkyl and 2-alkyl radicals of 9 to 15 carbon atoms having an average molecular weight of MA

$R^{2B}$ denotes 2-alkyl-l-alkyl radicals of 18 to 30 carbon atoms having an average molecular weight of MB

$R^{2C}$ denotes x-alkyl-y-alkyl radicals of 18 to 30 carbon atoms having an average molecular weight of MC where x signifies the position of the alkyl side chain on the alkyl main chain and y signifies the position of the bonding site of the alkyl main chain on the oxygen atom,

$R^{2D}$ denotes 1-alkoxyalkyl radicals of 18 to 30 carbon atoms having an average molecular weight of MD

$R^{2E}$ denotes x-alkylcarbonyloxy-y-alkyl radicals and/or x-alkoxycarbonyl-y-alkyl radicals of 27 to 51 carbon atoms having an average molecular weight of ME,
where x signifies the position of the alkylcarbonyloxy or alkoxycarbonyl substituent on the alkyl main chain and y signifies the position of the bonding site of the alkyl main chain on the oxygen atom,

$R^3$ comprises the radicals $R^{3F}$ and $R^{3G}$, where

$R^{3F}$ denotes 1,2-alkanediyl and/or 2-alkyl-1,3-alkanediyl of 10 to 16 carbon atoms having an average molecular weight of MF

$R^{3G}$ denotes 1,3-alkylalkanediyl of 18 to 30 carbon atoms having an average molecular weight of MG,

with OH-free ethers and esters of 18 to 45 carbon atoms and optionally alkanols of the formula $R^2OH$ and alkanediols of the formula $R^3(OH)_2$,
the $-OR^{2A}$ groups having a fraction of A[%] = 0 to 20% by weight,
the $-OR^{2B}$ groups having a fraction of B[%] = 0 to 40% by weight,
the $-OR^{2C}$ groups having a fraction of C[%] = 0 to 10% by weight,
the $-OR^{2D}$ groups having a fraction of D[%] = 0 to 20% by weight,
the $-OR^{2E}$ groups having a fraction of E[%] = 0 to 25% by weight,
the $(-O)_2R^{3F}$- groups having a fraction of F[%] = 0 to 7.5% by weight,
the $(-O)_2R^{3G}$- groups having a fraction of G[%] = 0 to 8% by weight,
and the OH-free ethers and esters having a fraction of H[%] = 0 to 45% by weight,
based on the sum total SUG of the weights of the OH-free ethers and esters, alkanols, alkanediols and $-OR^2$ and $(-O)_2R^3$ groups present in the mixture.

**3.** Mixtures as claimed in claims 1 and 2, wherein the fractions of the structures $-OR^{2A}$, $-OR^{2B}$, $-OR^{2C}$, $-OR^{2D}$, $-OR^{2E}$, $-OR^{3F}O$- and $-OR^{3G}O$- present in the building groups $-OR^2$ and $-OR^3$ and the fraction of ethers and esters are selected in such a way within the framework of the above-indicated limits that the number OHZ defined by equation (GL1)

$$OHZ=561 \cdot \left(\frac{A[\%]}{MA}+\frac{B[\%]}{MB}+\frac{C[\%]}{MC}+\frac{D[\%]}{MD}+\frac{E[\%]}{ME}\right)+112 \cdot \left(\frac{F[\%]}{MF}+\frac{G[\%]}{MG}\right) \quad (GL1)$$

where A[%], B[%], C[%], D[%], E[%], F[%] and G[%] are the abovementioned percentages of the structures present in the building groups $-OR^2$ and $-OR^3$, based on the sum total weight SUG, and MA, MB, MC, MD, ME, MF and MG are the molecular weights of said structures, is in the range from 65 to 160.

**4.** A process for preparing the mixtures of monoesters of the formulae I and II of claim 1 by reacting inner anhydrides of di- or tribasic carboxylic acids with alkanols of medium and/or relatively large chain length, which comprises

A) reacting inner anhydrides of the formula IV

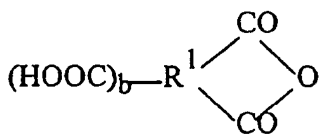

(IV)

where b is 0 or 1, and

$R^1$ is a di- or trivalent saturated or mono- or diunsaturated aliphatic or cycloaliphatic hydrocarbon radical of 2 to 6 carbon atoms or a di- or trivalent aromatic hydrocarbon radical of 6 carbon atoms, which radical may bear a sulfo group,

at from about 20 to not more than 120°C in an equivalent ratio of from 1 to 1 to 1 to 5 with a mixture of alcohols of the formula $R^2OH$, where

$R^2$ is predominantly branched, optionally hydroxyl-, alkoxy-, alkylcarbonyloxy- or alkoxycarbonyl-substituted alkyl of 9 to 51 carbon atoms,

alkanediols of the formula $(HO)_2R^3$, where

$R^3$ is alkanediyl of 10 to 30 carbon atoms,

and OH-free ethers and esters of 18 to 45 carbon atoms, and

B) to prepare mixtures according to the invention in which $R^1$ has a sulfo group from a sulfo-devoid anhydride (IV) conducting the preparation step A) using an anhydride (IV) whose $R^1$ radical is at least monounsaturated and then reacting the monoester mixture obtained according to section A), if appropriate after at least partial neutralization of its free carboxyl groups, in a conventional manner with a water-soluble sulfite, bisulfite or disulfite to add the sulfite or bisulfite to the double bond of the $R^1$ group to form the desired sulfo acid.

**5.** A process as claimed in claim 4, using mixtures of alcohols $R^2OH$ and diols $R^3(OH)_2$ comprising

from 0 to 20% by weight of $C_9$-$C_{15}$ alkanols
from 0 to 40% by weight of $C_{18}$-$C_{30}$ 2-alkyl alcohols
from 0 to 10% by weight of $C_{18}$-$C_{30}$ secondary alcohols
from 0 to 20% by weight of $C_{18}$-$C_{30}$ ether alcohols
from 0 to 25% by weight of $C_{27}$-$C_{51}$ ester alcohols
from 0 to 7.5% by weight of $C_{10}$-$C_{16}$ 1,2-diols
from 0 to 8% by weight of $C_{18}$-$C_{30}$ diols and
from 0 to 45% by weight of OH-free $C_{18}$-$C_{45}$ ethers and esters.

**6.** A process as claimed in claims 4 and 5, wherein the inner anhydrides of the formula IV are reacted with oxo thick oils as an alcohol component.

**7.** The use of the monoester mixtures of claim 1 as emulsifiers after partial or complete neutralization of the carboxylic acid groups.

**8.** The use of the monoester mixtures of claim 1 as assistants in leather manufacture.

**9.** A use as claimed in claim 7, wherein the monoester mixtures of claim 1 are used in combination with leather fatliquoring agents and/or leather hydrophobicizing agents.

**10.** Formulations including fatliquoring and/or hydrophobicizing substances required for fatliquoring and/or hydrophobicizing leather as well as an effective fraction of the monoester mixtures of claim 1.

## Revendications

**1.** Mélanges de monoesters d'acides carboxyliques dibasiques ou tribasiques des formules I et II :

$$(MOOC)_a\text{-}R^1\text{-}CO\text{-}OR^2 \qquad (I)$$

$$[(MOOC)_a\text{-}R^1\text{-}CO\text{-}O]_2R^3 \qquad (II)$$

dans lesquelles a représente les chiffres 1 ou 2, M représente de l'hydrogène ou un équivalent métallique, et

$R^1$ représente un radical d'hydrocarbure aliphatique ou cycloaliphatique divalent ou trivalent, saturé ou insaturé à une ou deux reprises, qui comporte 2 à 6 atomes de C et peut être substitué par un groupe acide sulfonique, ou un radical d'hydrocarbure aromatique divalent ou trivalent comportant 6 atomes de C,
$R^2$ représente un radical alkyle comportant 9 à 51 atomes de C, qui d'une manière prépondérante est ramifié et est éventuellement substitué par un groupe hydroxy, alcoxy, alkylcarbonyloxy ou alcoxycarbonyle, et
$R^3$ représente un radical alcanediyle comportant 10 à 30 atomes de C,

avec des éthers et esters exempts de groupes OH, comportant 18 à 45 atomes de C, et éventuellement des alcanols de la formule $R^2OH$ et des alcanediols de la formule $R^3(OH)_2$, la fraction des groupes $-OR^2$ allant jusqu'à 85 %, la fraction des groupes $(-O)_2R^3$ jusqu'à 16 % et la fraction des éthers et esters exempts de groupes OH jusqu'à 45 %, par rapport à la somme SUG des poids des éthers exempts de groupes OH, esters exempts de groupes OH, alcanols, alcanediols et groupes $-OR^2$ et $(-O)_2R^3$ contenus dans le mélange.

**2.** Mélanges suivant la revendication 1, **caractérisés en ce que**

$R^1$ représente un radical d'hydrocarbure aliphatique divalent saturé, comportant 2 à 4 atomes de C, qui peut être substitué par un groupe acide sulfonique,
ou un radical d'hydrocarbure aliphatique divalent, insaturé à une reprise, comportant 2 à 4 atomes de C,
ou un radical d'hydrocarbure cycloaliphatique divalent ou trivalent, saturé, comportant 6 atomes de C, qui peut être substitué par un groupe acide sulfonique,
ou un radical d'hydrocarbure cycloaliphatique divalent ou trivalent comportant 6 atomes de C, qui présente une ou éventuellement deux doubles liaisons,
ou un radical d'hydrocarbure aromatique divalent ou trivalent comportant 6 atomes de C,
$R^2$ comporte les radicaux $R^{2A}$, $R^{2B}$, $R^{2C}$, $R^{2D}$ et $R^{2E}$, où
$R^{2A}$ représente des radicaux alkyle(1) et alkyle(2) comportant 9 à 15 atomes de C, avec un poids moléculaire moyen de MA,
$R^{2B}$ représente des radicaux 2-alkyl-alkyle(1) comportant 18 à 30 atomes de C, avec un poids moléculaire moyen de MB,
$R^{2C}$ représente des radicaux x-alkyl-alkyle(y) comportant 18 à 30 atomes de C, avec un poids moléculaire moyen de MC,
x représentant la position de la chaîne latérale alkylique sur la chaîne principale alkylique et y représentant la position du site de liaison de la chaîne principale alkylique à l'atome d'oxygène,
$R^{2D}$ représente des radicaux alcoxyalkyle(1) comportant 18 à 30 atomes de C, avec un poids moléculaire moyen de MD,
$R^{2E}$ représente des radicaux x-alkylcarbonyloxy-alkyle(y) et/ou x-alcoxycarbonyl-alkyle(y) comportant 27 à 51 atomes de C, avec un poids moléculaire moyen de ME, où x représente la position des substituants alkylcarbonyloxy ou alcoxycarbonyle sur la chaîne principale alkylique et y représente la position du site de liaison de la chaîne principale alkylique à l'atome d'oxygène,
$R^3$ comporte les radicaux $R^{3F}$ et $R^{3G}$, où
$R^{3F}$ représente un radical alcanediyle(1,2) et/ou 2-alkyl-alcanediyle(1,3) comportant 10 à 16 atomes de C, avec un poids moléculaire moyen de MF,
$R^{3G}$ représente un radical alkyl-alcanediyle(1,3) comportant 18 à 30 atomes de C, avec un poids moléculaire moyen de MG,

avec des éthers et esters exempts de groupes OH et comportant 18 à 45 atomes de C, et éventuellement des

alcanols de la formule $R^2OH$ et des alcanediols de la formule $R^3(OH)_2$,
et **en ce que**, par rapport à la somme SUG des poids des éthers et esters exempts de groupes OH, alcanols, alcanediols et groupes $-OR^2$ et $(-O)_2R^3$ contenus dans le mélange,
les groupes $-OR^{2A}$ présentent une fraction de A[%] = 0 à 20 % en poids,
les groupes $-OR^{2B}$ une fraction de B[%] = 0 à 40 % en poids,
les groupes $-OR^{2C}$ une fraction de C[%] = 0 à 10 % en poids,
les groupes $-OR^{2D}$ une fraction de D[%] = 0 à 20 % en poids,
les groupes $-OR^{2E}$ une fraction de E[%] = 0 à 25 % en poids,
les groupes $(-O)_2R^{3F}$ une fraction de F[%] = 0 à 7,5 % en poids,
les groupes $(-O)_2R^{3G}$ une fraction de G[%] = 0 à 8 % en poids,
et les éthers et esters exempts de groupes OH une fraction de H[%] = 0 à 45 % en poids.

**3.** Mélanges suivant les revendications 1 et 2,
**caractérisés en ce que** les fractions des structures $-OR^{2A}$, $-OR^{2B}$, $-OR^{2C}$, $-OR^{2D}$, $-OR^{2E}$, $-OR^{3F}O-$ et $-OR^{3G}O-$ contenues dans les groupes de construction $-OR^2$ et $-OR^3$ et la fraction des éthers et esters sont choisies dans le cadre des limites indiquées ci-dessus de sorte que la grandeur OHZ définie par l'équation (GL1)

$$OHZ=561 \bullet \left(\frac{A[\%]}{MA}+\frac{B[\%]}{MB}+\frac{C[\%]}{MC}+\frac{D[\%]}{MD}+\frac{E[\%]}{ME}\right)+112\bullet\left(\frac{F[\%]}{MF}+\frac{G[\%]}{MG}\right) \quad (GL1)$$

dans laquelle A[%], B[%], C[%], D[%], E[%], F[%] et G[%] sont les fractions en % précitées des structures contenues dans les groupes de construction $-OR^2$ et $-OR^3$, par rapport au poids cumulé SUG, et MA, MB, MC, MD, ME, MF et MG sont les poids moléculaires des structures considérées, soit de l'ordre de 65 à 160.

**4.** Procédé de préparation des mélanges de monoesters des formules I et II de la revendication 1, par réaction d'anhydrides internes d'acides carboxyliques dibasiques ou tribasiques avec des alcanols de longueur de chaîne moyenne et/ou grande, **caractérisé en ce qu'**on fait réagir

A) des anhydrides internes de la formule IV :

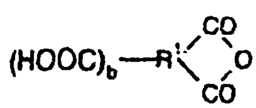 (IV)

dans laquelle b représente les chiffres 0 ou 1, et

$R^1$ représente un radical d'hydrocarbure aliphatique ou cycloaliphatique divalent ou trivalent, saturé ou insaturé à une ou deux reprises, comportant 2 à 6 atomes de C, ou un radical d'hydrocarbure aromatique divalent ou trivalent comportant 6 atomes de C, qui peut porter un groupe sulfonique,

à une température d'environ 20 à au maximum 120°C, dans un rapport équivalent de 1/1 à 1/5, avec un mélange
d'alcools de la formule $R^2OH$, dans laquelle

$R^2$ représente un radical alkyle comportant 9 à 51 atomes de C, qui est d'une manière prépondérante ramifié et est éventuellement substitué par un groupe hydroxy, alcoxy, alkylcarbonyloxy ou alcoxycarbonyle,

d'alcanediols de la formule $(HO)_2R^3$, dans laquelle

$R^3$ représente un radical alcanediyle comportant 10 à 30 atomes de C, et

d'éthers et esters exempts de groupes OH comportant 18 à 45 atomes de C, et
B) dans le cas où des mélanges suivant l'invention, dans lesquels $R^1$ représente un groupe sulfonique, doivent

être préparés à partir d'un anhydride (IV) exempt de groupes acide sulfonique, on met en oeuvre pour l'étape de préparation A), un anhydride IV dont le radical $R^1$ est insaturé au moins à une reprise et on fait alors réagir le mélange de monoesters obtenu suivant l'étape A), éventuellement après au moins une neutralisation partielle de ses groupes carboxyle libres, d'une manière connue en soi avec un sulfite, hydrogénosulfite ou disulfite soluble dans l'eau, avec addition du sulfite ou de l'hydrogénosulfite sur la double liaison du groupe $R^1$, avec formation de l'acide sulfonique souhaité.

**5.** Procédé suivant la revendication 4, **caractérisé en ce qu'** on met en oeuvre des mélanges d'alcools $R^2OH$ et de diols $R^3(OH)_2$ qui comportent

0 à 20 % en poids d'alcanols en $C_9$-$C_{15}$,
0 à 40 % en poids d'alcools 2-alkyliques en $C_{18}$-$C_{30}$,
0 à 10 % en poids d'alcools secondaires en $C_{18}$-$C_{30}$,
0 à 20 % en poids d'étheralcools en $C_{18}$-$C_{30}$,
0 à 25 % en poids d' esteralcools en $C_{27}$-$C_{51}$,
0 à 7,5 % en poids de 1,2-diols en $C_{10}$-$C_{l6}$,
0 à 8 % en poids de diols en $C_{18}$-$C_{30}$,
0 à 45 % en poids d'éthers en $C_{18}$-$C_{45}$ et d'esters en $C_{18}$-$C_{45}$ exempts de groupes OH.

**6.** Procédé suivant les revendications 4 et 5,
**caractérisé en ce que** les anhydrides internes de la formule IV sont mis à réagir avec des huiles épaisses oxo comme composants alcool.

**7.** Utilisation des mélanges de monoesters de la revendication 1, après neutralisation ou neutralisation partielle des groupes acide carboxylique, comme agents émulsionnants.

**8.** Utilisation des mélanges de monoesters de la revendication 1, comme adjuvants dans la fabrication des cuirs.

**9.** Utilisation suivant la revendication 7, **caractérisée en ce que** les mélanges de monoesters de la revendication 1 sont mis en oeuvre en combinaison avec des agents de graissage de cuir et/ou des agents rendant les cuirs hydrophobes.

**10.** Compositions qui, outre une fraction active des mélanges de monoesters de la revendication 1, contiennent des substances graissantes et/ou rendant hydrophobes requises pour graisser et/ou rendre hydrophobes des cuirs.